# EUROPEAN PATENT APPLICATION

(11) **EP 4 559 895 A1**
(43) Date of publication of application: **28.05.2025**
(21) Application number: 23842693.6
(22) Date of filing: 31.05.2023
(51) Int. Cl.: C07C 237/08, A61K 9/14, A61K 31/7088, A61K 31/7105, A61K 31/711, A61K 47/18, A61K 48/00, C11B 11/00

(54) **NEUTRAL LIPID AND LIPID NANOPARTICLE**

(30) Priority: 19.07.2022 JP 2022115017
(71) Applicant: National University Corporation Hokkaido University, Hokkaido 060-0808 (JP)
(72) Inventor: SATO, Yusuke, Sapporo-shi, Hokkaido 060-0808 (JP); HARASHIMA, Hideyoshi, Sapporo-shi, Hokkaido 060-0808 (JP); SUZUKI, Yuichi, Sapporo-shi, Hokkaido 060-0808 (JP)
(74) Representative: Müller-Boré & Partner Patentanwälte PartG mbB
(86) International application number: PCT/JP2023/020375
(87) International publication number: WO 2024/018761

(57) **Abstract**

The present invention provides: a neutral lipid capable of suppressing the phase transition of endosomal membranes caused by phosphatidylcholine from being inhibited; and a lipid nanoparticle containing the neutral lipid. The present invention pertains to an ionic neutral lipid comprising a compound represented by general formula (I). [In formula (I), R¹ is a C1-22 hydrocarbon group; three R¹ groups in one molecule may be the same as or different from each other; a1 and a2 are each independently an integer of 0 to 4; b1 and b2 are 0 or 1, satisfying b1+b2=1; R² and R³ are each independently a hydrogen atom or a C1-3 alkyl group; and R4 is an anionic group.]

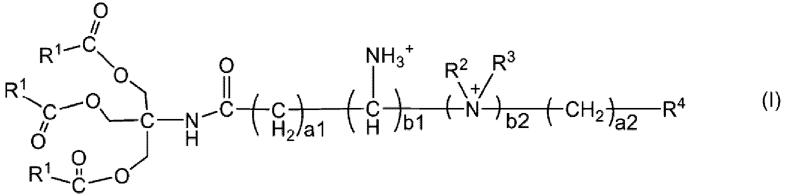

## Description

### Technical Field

The present invention relates to a neutral lipid having a hydrophobic chain and a hydrophilic group, and a lipid nanoparticle containing the neutral lipid.

This application claims priority to Japanese Patent Application No. 2022-115017 filed in Japan on July 19, 2022, the contents of which are incorporated herein by reference.

### Background Art

Lipid nanoparticles (LNPs) are used as carriers to encapsulate fat-soluble drugs and nucleic acids such as siRNA (short interfering RNA) and mRNA and deliver them to target cells. For example, as lipid nanoparticles that serve as carriers for efficiently delivering nucleic acids such as siRNA into target cells, lipid nanoparticles have been reported that contain pH-sensitive cationic lipids, which are electrically neutral at physiological pH and change to cationic in a weakly acidic pH environment such as an endosome, as constituent lipids (Patent Literature 1). Lipid nanoparticles carrying a positive charge in the blood interact non-specifically with plasma proteins and are rapidly cleared from the blood by the reticuloendothelial system. In contrast, lipid nanoparticles containing pH-sensitive cationic lipids as constituent lipids are not charged under neutral conditions in the blood, but are positively charged under weakly acidic conditions in endosomes and can efficiently escape from endosomes.

As a pH-sensitive cationic lipid, for example, Jayaraman et al. developed DLin-MC3-DMA and achieved an ED₅₀ of 0.005 mg siRNA/kg in factor VII (F7) knockdown in mouse liver (Non-patent Literature 1). The present inventors have also previously developed their own pH-sensitive cationic lipids YSK05 and YSK13-C3, achieving ED₅₀s of 0.06 and 0.015 mg siRNA/kg, respectively, in F7 knockdown (Non-patent Literatures 2-4). The present inventors have further developed compounds having two hydrocarbon chains and one hydrophilic group, such as 7-(4-(diisopropylamino)butyl)-7-hydroxytridecane-1,13-diyl dioleate (CL4H6), as pH-sensitive cationic lipids (Patent Literature 1).

Lipid nanoparticles are taken up into cells by endocytosis. Therefore, it is essential for improving the efficiency of uptake of lipid nanoparticles into cells that the lipid nanoparticles easily fuse with the endosomal membrane. On the other hand, phosphatidylcholine (PC), the main constituent lipid of the endosomal membrane, takes a cylindrical shape and thus stabilizes the lamellar (L) phase of the lipid bilayer (planar membrane) and strongly inhibits the transition to the inverse hexagonal (HII) phase, which is essential for membrane fusion. For example, it is traditionally known that DOPE, a constituent lipid commonly used in lipid nanoparticles, takes a cone shape and thus promotes the transition from the lamellar phase to the inverse hexagonal phase, but its action is greatly inhibited by phosphatidylcholine (Non-patent Literature 5).

On the other hand, as a method for producing lipid nanoparticles encapsulating nucleic acids and the like, there is a method based on the principle of alcohol dilution using a flow path. For example, it has been reported that lipid nanoparticles having a diameter of about 30 nm can be reproducibly produced by using a microchannel with a built-in three-dimensional micromixer capable of achieving instantaneous mixing of two liquids (Non-patent Literature 6). It has also been reported that by using a simple two-dimensional flow path structure in which baffles of a certain width are arranged alternately from both sides in a micro-sized flow path through which a raw material solution flows, it is possible to form a nano-sized lipid particle formation system with higher particle size controllability than the conventional flow path structure using a three-dimensional mixer (Patent Literature 2). Recently, these methods for producing nanoparticle formulations have been mainly employed for the production of lipid nanoparticles (LNPs) loaded with fat-soluble drugs and nucleic acids such as siRNA (short interfering RNA) or mRNA.

### Citation List

### Patent Literatures

Patent Literature 1: International Publication No. WO 2018/230710
Patent Literature 2: International Publication No. WO 2018/190423

### Non Patent Literatures

Non Patent Literature 1: Jayaraman et al., Angewandte Chemie International Edition, 2012, vol. 51, pp. 8529-8533.
Non Patent Literature 2: Watanabe et al., Scientific Reports, 2014, 4:4750, DOI: 10.1038/srep04750.
Non Patent Literature 3: Yamamoto et al., Journal of Hepatology, 2016, vol. 64, pp. 547-555.
Non Patent Literature 4: Sato et al., Molecular Therapy, 2016, vol. 24, pp. 788-795.
Non Patent Literature 5: Cullis et al., Biochimica et Biophysica Acta, 1979, vol. 559, pp. 399-420.
Non Patent Literature 6: Leung et al., Journal of Physical Chemistry C Nanomater Interfaces, 2012, vol. 116(34), pp. 18440-18450.

### Summary of Invention

### Problems to be solved by the invention

In the uptake of lipid nanoparticles containing pH-sensitive cationic lipids into cells, the phase transition driven by the interaction between the pH-sensitive cationic lipid in the lipid nanoparticle and the anionic lipid in the endosomal membrane is important; however, this phase transition is inhibited by neutral phospholipids such as phosphatidylcholine. Therefore, there is a need for the development of constituent lipids of lipid nanoparticles that can suppress the inhibition of phase transition by this neutral phospholipid.

An object of the present invention is to provide a neutral lipid capable of suppressing the inhibition of phase transition of the endosomal membrane by phosphatidylcholine, and a lipid nanoparticle containing the neutral lipid.

### Means for solution of the problems

The present inventors have found that a compound in which a hydrocarbon chain is bonded to each of the three hydroxyl groups of tris(hydroxymethyl)aminomethane (CAS number: 77-86-1) (hereinafter abbreviated as "Tris") and a hydrophilic group having a cationic moiety and an anionic group at the terminal thereof is bonded to the remaining amino group is a suitable ionic neutral lipid as a constituent lipid of lipid nanoparticles, thereby completing the present invention.

That is, the present invention provides the following ionic neutral lipids and the like.
[1] An ionic neutral lipid comprising a compound represented by the following formula (I): 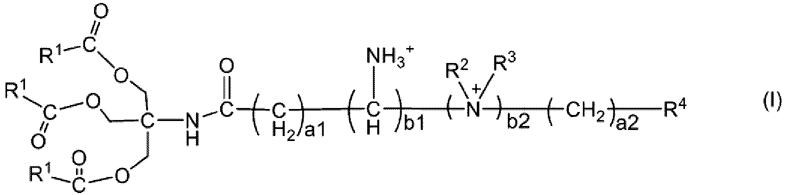 wherein, in formula (I), R¹ is a hydrocarbon group having 1 to 22 carbon atoms; three R¹ groups in one molecule may be the same as or different from each other; a1 and a2 are each independently an integer of 0 to 4; b1 and b2 are 0 or 1 satisfying b1+b2=1; R² and R³ are each independently a hydrogen atom or an alkyl group having 1 to 3 carbon atoms; and R⁴ is an anionic group.
[2] The ionic neutral lipid according to [1], wherein R⁴ in the general formula (I) is a carboxylic acid group, a sulfonic acid group, a sulfate ester group, or a phosphate group.
[3] The ionic neutral lipid according to [1] or [2], wherein the three R¹ groups in one molecule are all the same.
[4] The ionic neutral lipid according to any one of [1] to [3], wherein R¹ is a hydrocarbon group having 15 to 22 carbon atoms.
[5] The ionic neutral lipid according to any one of [1] to [4], which is a compound represented by any one of the following general formulas (Z-9) to (Z-15), wherein R¹ is the same as in the general formula (I). 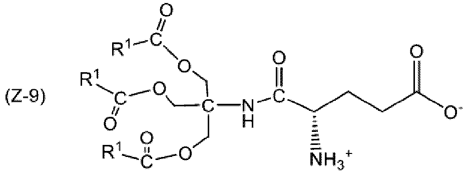 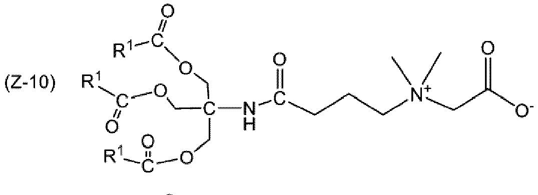 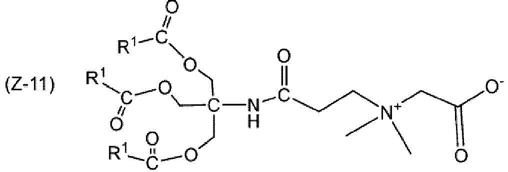 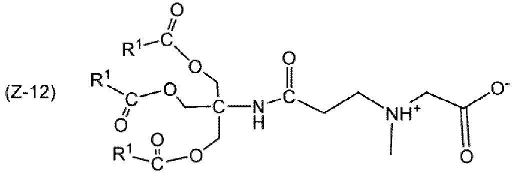 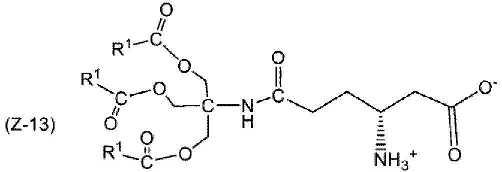 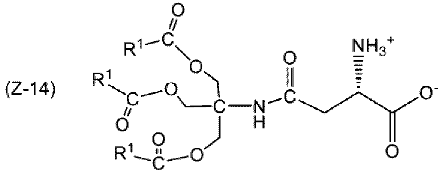 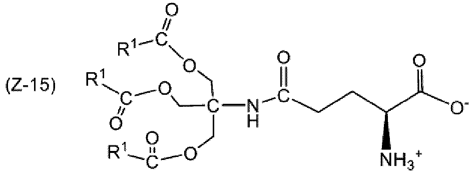
[6] The ionic neutral lipid according to any one of [1] to [5], which is
   (S)-4-amino-5-((1,3-bis(oleoyloxy)-2-((oleoyloxy)methyl)propan-2-yl)amino)-5-oxopen tanoic acid,
   2-((4-((1,3-bis(oleoyloxy)-2-((oleoyloxy)methyl)propan-2-yl)amino)-4-oxobutyl)dimeth ylammonio)acetate,
   2-((3-((1,3-bis(oleoyloxy)-2-((oleoyloxy)methyl)propan-2-yl)amino)-3-oxopropyl)dime thylammonio)acetate,
   2-((3-((1,3-bis(oleoyloxy)-2-((oleoyloxy)methyl)propan-2-yl)amino)-3-oxopropyl)(met hyl)ammonio)acetate,
   (S)-3-amino-4-((1,3-bis(oleoyloxy)-2-((oleoyloxy)methyl)propan-2-yl)amino)-4-oxobut anoic acid, N4-(1,3-bis(oleoyloxy)-2-((oleoyloxy)methyl)propan-2-yl)-L-asparagine, or N5-(1,3-bis(oleoyloxy)-2-((oleoyloxy)methyl)propan-2-yl)-L-glutamine.
[7] An ionic neutral lipid comprising a Tris skeleton, wherein
   a fatty acid residue is bonded to each of three oxygen atoms of the Tris skeleton, the fatty acid residue has 1 to 22 carbon atoms in a hydrocarbon chain moiety thereof and may have one or more unsaturated bonds, and the three fatty acid residues in one molecule may be the same group or two or more different groups, and
   an anionic group is linked to one nitrogen atom of the Tris skeleton via a divalent linking group having an ammonium cationic group or a quaternary ammonium cationic nitrogen atom.
[8] A lipid nanoparticle comprising the ionic neutral lipid according to any one of [1] to [7].
[9] The lipid nanoparticle according to [8], further comprising a sterol and a polyalkylene glycol-modified lipid.
[10] The lipid nanoparticle according to [8] or [9], further comprising a pH-sensitive cationic lipid having a pKa of 6.6 or less.
[11] The lipid nanoparticle according to any one of [8] to [10], containing a nucleic acid.
[12] The lipid nanoparticle according to [11], wherein the nucleic acid is siRNA, mRNA, or plasmid DNA.
[13] A pharmaceutical composition comprising the lipid nanoparticle containing the ionic neutral lipid according to any one of [1] to [7] as an active ingredient.
[14] A pharmaceutical composition comprising, as an active ingredient, the lipid nanoparticle containing the ionic neutral lipid according to any one of [1] to [7] and a nucleic acid.
[15] The pharmaceutical composition according to [13] or [14], which is used for gene therapy.
[16] A method for expressing a foreign gene, comprising administering to a subject animal (excluding human), the lipid nanoparticle according to [11], which encapsulates a foreign gene of interest to be expressed in a target cell, and expressing the foreign gene in the target cell of the subject animal.

### Effects of Invention

The ionic neutral lipid according to the present invention acts suppressively on the stabilizing action of phosphatidylcholine on the lipid bilayer. Therefore, the ionic neutral lipid is suitable as a constituent lipid of lipid nanoparticles for uptake into cells, and is particularly useful as a constituent lipid of lipid nanoparticles used as an active ingredient of a pharmaceutical.

### Brief Description of Drawings

Fig. 1 illustrates ³¹P NMR spectral data of DOPC/DOPE membrane (Fig. 1(A)) and DOPC/TOT-10 membrane (Fig. 1(B)) in Example 8.
Fig. 2 illustrates a diagram showing the results of measurement of Fluc activity (RLU/mg protein) in the liver and spleen of mice administered with Fluc mRNA-loaded lipid nanoparticles in Example 9.
Fig. 3 illustrates a diagram showing the results of measurement of Fluc activity (RLU/mg protein) in the liver of mice administered with Fluc mRNA-loaded lipid nanoparticles in Example 10.
Fig. 4 illustrates a diagram showing the results of measurement of Fluc activity (RLU/mg protein) in the quadriceps femoris muscle of mice administered with Fluc mRNA-loaded lipid nanoparticles in Example 11.
Fig. 5 illustrates a diagram showing the results of measurement of Fluc activity (RLU/mg protein) in the brain of mice administered with Fluc mRNA-loaded lipid nanoparticles in Example 12.
Fig. 6 illustrates a diagram showing the results of measurement of Fluc activity (RLU/mg protein) in the liver and spleen of mice administered with Fluc mRNA-loaded lipid nanoparticles in Example 13.
Fig. 7 illustrates a diagram showing the measurement results of ionization degree (%) at each pH of lipid nanoparticles prepared from CL4F6, TOT-9 to TOT-15, or DSPC, cholesterol, and PEG-DMG in Example 14.
Fig. 8 illustrates a diagram showing the results of measurement of hemolytic activity of lipid nanoparticles prepared from CL4F6, TOT-9 to TOT-15, or DSPC, cholesterol, and PEG-DMG in Example 14.
Fig. 9 illustrates a diagram showing the measurement results of ionization degree (%) at each pH of lipid nanoparticles prepared using CL4F6, ALC-0315, MC3, SM-102, CL15F6, or CL15H6 as a cationic lipid and TOT-15 or DSPC as an ionic neutral lipid in Example 14.
Fig. 10 illustrates a diagram showing the results of measurement of hemolytic activity of lipid nanoparticles prepared using CL4F6, ALC-0315, MC3, SM-102, CL15F6, or CL15H6 as a cationic lipid and TOT-15 or DSPC as an ionic neutral lipid in Example 14.
Fig. 11 illustrates a diagram showing the results of measurement of Fluc mRNA amount in the liver of mice administered with Fluc mRNA-loaded lipid nanoparticles in Example 15.
Fig. 12 illustrates a diagram showing the results of measurement of Fluc activity (RLU/mg protein) in the liver of mice administered with Fluc mRNA-loaded lipid nanoparticles in Example 15.
Fig. 13 illustrates a diagram showing the results of measurement of knockdown efficiency against TTR in mice administered with TTR-targeted genome editing lipid nanoparticles loaded with Cas9-mRNA and TTR-gRNA in Example 16.
Fig. 14 illustrates a diagram showing the results of time-course measurement of average particle size and encapsulation rate during storage at 4°C of Fluc mRNA-loaded lipid nanoparticles prepared from CL4F6, TOT-15 or DSPC, cholesterol, and PEG-DMG in Example 17.
Fig. 15 illustrates a diagram showing the results of measurement of Fluc activity (RLU/mg protein) in the liver of mice administered with Fluc mRNA-loaded lipid nanoparticles immediately after production or after storage at 4°C for 4 weeks in Example 17.
Fig. 16 illustrates microscopic images observing EGFP expression in the liver of EGFP mRNA-loaded lipid nanoparticles prepared from CL4F6, TOT-15 or DSPC, cholesterol, and PEG-DMG in Example 18.

### Description of Embodiments

Hereinafter, embodiments of the present invention will be specifically described. In this specification, "X1 to X2 (X1 and X2 are real numbers satisfying X1 < X2)" means "X1 or more and X2 or less." Also, "C_{X3-X4} (X3 and X4 are integers satisfying X3 < X4)" means "having X3 or more and X4 or less carbon atoms."

### [Ionic Neutral Lipid]

The ionic neutral lipid according to the present invention is a compound in which a hydrocarbon chain is bonded to each of the three hydroxyl groups of Tris, and an anionic group containing a cationic moiety is bonded to the amino group of Tris. It is a compound having three hydrocarbon chains and a hydrophilic moiety and has a high affinity for lipids such as phospholipids due to the similarity of its structure, and is suitable as a constituent lipid of lipid nanoparticles.

As an example of the ionic neutral lipid according to the present invention, a compound in which a fatty acid residue is bonded to each of the three oxygen atoms in a Tris skeleton, and an anionic group is linked to one nitrogen atom in the Tris skeleton via a linking group having an ammonium cationic group or a quaternary ammonium cationic nitrogen atom can be mentioned. Here, the term "Tris skeleton" means a structure in which one hydrogen atom is removed from each of the three hydroxyl groups and the amino group of tris(hydroxymethyl)aminomethane. The three fatty acid residues bonded to the Tris skeleton have 1 to 22 carbon atoms in the hydrocarbon chain moiety and may have one or more unsaturated bonds. Further, a plurality of fatty acid residues in one molecule may be the same group or two or more different groups. The ionic neutral lipid according to the present invention preferably has all of the plurality of fatty acid residues in one molecule being the same group. As the anionic group linked to one nitrogen atom of the Tris skeleton via a divalent linking group, the groups listed in R⁴ below can be used. The divalent linking group linking one nitrogen atom of the Tris skeleton to the anionic group is not particularly limited as long as it is a divalent group in which an ammonium cationic group is inserted into the side chain, or a group in which a quaternary ammonium cationic nitrogen atom is inserted between carbon atoms constituting the main chain, and for example, a group in which one hydrogen atom bonded to a carbon atom of an alkylene group having 1 to 6 carbon atoms is substituted with an ammonium cationic group, or a group in which a quaternary ammonium cationic nitrogen atom is inserted between carbon atoms of an alkylene group having 1 to 6 carbon atoms is preferable. As the ammonium cationic group and the quaternary ammonium cationic nitrogen atom, the groups listed below can be used.

An example of the ionic neutral lipid according to the present invention is a compound represented by the following general formula (I):

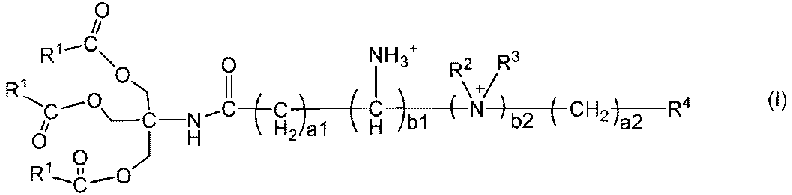

In the general formula (I), R¹ is a hydrocarbon group having 1 to 22 carbon atoms (C₁₋₂₂ hydrocarbon group). The C₁₋₂₂ hydrocarbon group may be linear or branched. The hydrocarbon group may be an alkyl group or an alkenyl group. The alkenyl group may be a group having one unsaturated bond, two unsaturated bonds, or three unsaturated bonds. R¹ is preferably a hydrocarbon group having 15 to 22 carbon atoms (C₁₅₋₂₂ hydrocarbon group).

When R¹ is a C₁₋₂₂ alkyl group, examples of the alkyl group include:
methyl group, ethyl group, n-propyl group, isopropyl group, n-butyl group, isobutyl group, sec-butyl group, tert-butyl group;
n-pentyl group, 1-methylbutyl group, 2-methylbutyl group, 3-methylbutyl group, 1-ethylpropyl group, 1,1-dimethylpropyl group, 2,2-dimethylpropyl group;
n-hexyl group, 1-methylpentyl group, 2-methylpentyl group, 3-methylpentyl group, 4-methylpentyl group, 1-ethylbutyl group, 1,1-dimethylbutyl group, 2,2-dimethylbutyl group, 3,3-dimethylbutyl group, 1,2-dimethylbutyl group, 1-methyl-2,2-dimethylbutyl group;
n-heptyl group, 1-methylhexyl group, 2-methylhexyl group, 3-methylhexyl group, 4-methylhexyl group, 5-methylhexyl group, 1-ethylpentyl group, 1,1-dimethylpentyl group, 2,2-dimethylpentyl group, 3,3-dimethylpentyl group, 4,4-dimethylpentyl group, 1-methyl-3,3-dimethylbutyl group, 2-methyl-3,3-dimethylbutyl group;
n-octyl group, 1-methylheptyl group, 2-methylheptyl group, 3-methylheptyl group, 4-methylheptyl group, 5-methylheptyl group, 6-methylheptyl group, 1-ethylhexyl group, 1,1-dimethylhexyl group, 2,2-dimethylhexyl group, 3,3-dimethylhexyl group, 4,4-dimethylhexyl group, 5,5-dimethylhexyl group, 1-methyl-4,4-dimethylpentyl group, 2-methyl-4,4-dimethylpentyl group, 3-methyl-4,4-dimethylpentyl group;
n-nonyl group, 1-methyloctyl group, 2-methyloctyl group, 3-methyloctyl group, 4-methyloctyl group, 5-methyloctyl group, 6-methyloctyl group, 7-methyloctyl group, 1-ethylheptyl group, 1,1-dimethylheptyl group, 2,2-dimethylheptyl group, 3,3-dimethylheptyl group, 4,4-dimethylheptyl group, 5,5-dimethylheptyl group, 6,6-dimethylheptyl group, 1-methyl-5,5-dimethylhexyl group, 2-methyl-5,5-dimethylhexyl group, 3-methyl-5,5-dimethylhexyl group, 4-methyl-5,5-dimethylhexyl group;
n-decyl group, 1-methylnonyl group, 2-methylnonyl group, 3-methylnonyl group, 4-methylnonyl group, 5-methylnonyl group, 6-methylnonyl group, 7-methylnonyl group, 8-methylnonyl group, 1-ethyloctyl group, 1,1-dimethyloctyl group, 2,2-dimethyloctyl group, 3,3-dimethyloctyl group, 4,4-dimethyloctyl group, 5,5-dimethyloctyl group, 6,6-dimethyloctyl group, 7,7-dimethyloctyl group, 1-methyl-6,6-dimethylheptyl group, 2-methyl-6,6-dimethylheptyl group, 3-methyl-6,6-dimethylheptyl group, 4-methyl-6,6-dimethylheptyl group, 5-methyl-6,6-dimethylheptyl group;
n-undecyl group, 1-methyldecyl group, 2-methyldecyl group, 3-methyldecyl group, 4-methyldecyl group, 5-methyldecyl group, 6-methyldecyl group, 7-methyldecyl group, 8-methyldecyl group, 9-methyldecyl group, 1-ethylnonyl group, 1,1-dimethylnonyl group, 2,2-dimethylnonyl group, 3,3-dimethylnonyl group, 4,4-dimethylnonyl group, 5,5-dimethylnonyl group, 6,6-dimethylnonyl group, 7,7-dimethylnonyl group, 8,8-dimethylnonyl group, 1-methyl-7,7-dimethyloctyl group, 2-methyl-7,7-dimethyloctyl group, 3-methyl-7,7-dimethyloctyl group, 4-methyl-7,7-dimethyloctyl group, 5-methyl-7,7-dimethyloctyl group, 6-methyl-7,7-dimethyloctyl group;
n-dodecyl group, 1-methylundecyl group, 2-methylundecyl group, 3-methylundecyl group, 4-methylundecyl group, 5-methylundecyl group, 6-methylundecyl group, 7-methylundecyl group, 8-methylundecyl group, 9-methylundecyl group, 10-methylundecyl group, 1-ethyldecyl group, 1,1-dimethyldecyl group, 2,2-dimethyldecyl group, 3,3-dimethyldecyl group, 4,4-dimethyldecyl group, 5,5-dimethyldecyl group, 6,6-dimethyldecyl group, 7,7-dimethyldecyl group, 8,8-dimethyldecyl group, 9,9-dimethyldecyl group, 1-methyl-8,8-dimethylnonyl group, 2-methyl-8,8-dimethylnonyl group, 3-methyl-8,8-dimethylnonyl group, 4-methyl-8,8-dimethylnonyl group, 5-methyl-8,8-dimethylnonyl group, 6-methyl-8,8-dimethylnonyl group, 7-methyl-8,8-dimethylnonyl group;
n-tridecyl group, 1-methyldodecyl group, 2-methyldodecyl group, 3-methyldodecyl group, 4-methyldodecyl group, 5-methyldodecyl group, 6-methyldodecyl group, 7-methyldodecyl group, 8-methyldodecyl group, 9-methyldodecyl group, 10-methyldodecyl group, 11-methyldodecyl group, 1-ethylundecyl group, 1,1-dimethylundecyl group, 2,2-dimethylundecyl group, 3,3-dimethylundecyl group, 4,4-dimethylundecyl group, 5,5-dimethylundecyl group, 6,6-dimethylundecyl group, 7,7-dimethylundecyl group, 8,8-dimethylundecyl group, 9,9-dimethylundecyl group, 10,10-dimethylundecyl group, 1-methyl-9,9-dimethyldecyl group, 2-methyl-9,9-dimethyldecyl group, 3-methyl-9,9-dimethyldecyl group, 4-methyl-9,9-dimethyldecyl group, 5-methyl-9,9-dimethyldecyl group, 6-methyl-9,9-dimethyldecyl group, 7-methyl-9,9-dimethyldecyl group, 8-methyl-9,9-dimethyldecyl group;
n-tetradecyl group, 1-methyltridecyl group, 2-methyltridecyl group, 3-methyltridecyl group, 4-methyltridecyl group, 5-methyltridecyl group, 6-methyltridecyl group, 7-methyltridecyl group, 8-methyltridecyl group, 9-methyltridecyl group, 10-methyltridecyl group, 11-methyltridecyl group, 12-methyltridecyl group, 1-ethyldodecyl group, 1,1-dimethyldodecyl group, 2,2-dimethyldodecyl group, 3,3-dimethyldodecyl group, 4,4-dimethyldodecyl group, 5,5-dimethyldodecyl group, 6,6-dimethyldodecyl group, 7,7-dimethyldodecyl group, 8,8-dimethyldodecyl group, 9,9-dimethyldodecyl group, 10,10-dimethyldodecyl group, 11,11-dimethyldodecyl group, 1-methyl-10,10-dimethylundecyl group, 2-methyl-10,10-dimethylundecyl group, 3-methyl-10,10-dimethylundecyl group, 4-methyl-10,10-dimethylundecyl group, 5-methyl-10,10-dimethylundecyl group, 6-methyl-10,10-dimethylundecyl group, 7-methyl-10,10-dimethylundecyl group, 8-methyl-10,10-dimethylundecyl group, 9-methyl-10,10-dimethylundecyl group;
n-pentadecyl group, 1-methyltetradecyl group, 2-methyltetradecyl group, 3-methyltetradecyl group, 4-methyltetradecyl group, 5-methyltetradecyl group, 6-methyltetradecyl group, 7-methyltetradecyl group, 8-methyltetradecyl group, 9-methyltetradecyl group, 10-methyltetradecyl group, 11-methyltetradecyl group, 12-methyltetradecyl group, 13-methyltetradecyl group, 1-ethyltridecyl group, 1,1-dimethyltridecyl group, 2,2-dimethyltridecyl group, 3,3-dimethyltridecyl group, 4,4-dimethyltridecyl group, 5,5-dimethyltridecyl group, 6,6-dimethyltridecyl group, 7,7-dimethyltridecyl group, 8,8-dimethyltridecyl group, 9,9-dimethyltridecyl group, 10,10-dimethyltridecyl group, 11,11-dimethyltridecyl group, 12,12-dimethyltridecyl group, 1-methyl-11,11-dimethyldodecyl group, 2-methyl-11,11-dimethyldodecyl group, 3-methyl-11,11-dimethyldodecyl group, 4-methyl-11,11-dimethyldodecyl group, 5-methyl-11,11-dimethyldodecyl group, 6-methyl-11,11-dimethyldodecyl group, 7-methyl-11,11-dimethyldodecyl group, 8-methyl-11,11-dimethyldodecyl group, 9-methyl-11,11-dimethyldodecyl group, 10-methyl-11,11-dimethyldodecyl group;
n-hexadecyl group, 1-methylpentadecyl group, 2-methylpentadecyl group, 3-methylpentadecyl group, 4-methylpentadecyl group, 5-methylpentadecyl group, 6-methylpentadecyl group, 7-methylpentadecyl group, 8-methylpentadecyl group, 9-methylpentadecyl group, 10-methylpentadecyl group, 11-methylpentadecyl group, 12-methylpentadecyl group, 13-methylpentadecyl group, 14-methylpentadecyl group;
n-heptadecyl group, 1-methylhexadecyl group, 2-methylhexadecyl group, 3-methylhexadecyl group, 4-methylhexadecyl group, 5-methylhexadecyl group, 6-methylhexadecyl group, 7-methylhexadecyl group, 8-methylhexadecyl group, 9-methylhexadecyl group, 10-methylhexadecyl group, 11-methylhexadecyl group, 12-methylhexadecyl group, 13-methylhexadecyl group, 14-methylhexadecyl group, 15-methylhexadecyl group;
n-octadecyl group, 1-methylheptadecyl group, 2-methylheptadecyl group, 3-methylheptadecyl group, 4-methylheptadecyl group, 5-methylheptadecyl group, 6-methylheptadecyl group, 7-methylheptadecyl group, 8-methylheptadecyl group, 9-methylheptadecyl group, 10-methylheptadecyl group, 11-methylheptadecyl group, 12-methylheptadecyl group, 13-methylheptadecyl group, 14-methylheptadecyl group, 15-methylheptadecyl group, 16-methylheptadecyl group;
n-nonadecyl group, 1-methyloctadecyl group, 2-methyloctadecyl group, 3-methyloctadecyl group, 4-methyloctadecyl group, 5-methyloctadecyl group, 6-methyloctadecyl group, 7-methyloctadecyl group, 8-methyloctadecyl group, 9-methyloctadecyl group, 10-methyloctadecyl group, 11-methyloctadecyl group, 12-methyloctadecyl group, 13-methyloctadecyl group, 14-methyloctadecyl group, 15-methyloctadecyl group, 16-methyloctadecyl group, 17-methyloctadecyl group;
n-icosyl group, 1-methylnonadecyl group, 2-methylnonadecyl group, 3-methylnonadecyl group, 4-methylnonadecyl group, 5-methylnonadecyl group, 6-methylnonadecyl group, 7-methylnonadecyl group, 8-methylnonadecyl group, 9-methylnonadecyl group, 10-methylnonadecyl group, 11-methylnonadecyl group, 12-methylnonadecyl group, 13-methylnonadecyl group, 14-methylnonadecyl group, 15-methylnonadecyl group, 16-methylnonadecyl group, 17-methylnonadecyl group, 18-methylnonadecyl group;
n-henicosyl group, 1-methylicosyl group, 2-methylicosyl group, 3-methylicosyl group, 4-methylicosyl group, 5-methylicosyl group, 6-methylicosyl group, 7-methylicosyl group, 8-methylicosyl group, 9-methylicosyl group, 10-methylicosyl group, 11-methylicosyl group, 12-methylicosyl group, 13-methylicosyl group, 14-methylicosyl group, 15-methylicosyl group, 16-methylicosyl group, 17-methylicosyl group, 18-methylicosyl group, 19-methylicosyl group;
n-docosyl group, 1-methylhenicosyl group, 2-methylhenicosyl group, 3-methylhenicosyl group, 4-methylhenicosyl group, 5-methylhenicosyl group, 6-methylhenicosyl group, 7-methylhenicosyl group, 8-methylhenicosyl group, 9-methylhenicosyl group, 10-methylhenicosyl group, 11-methylhenicosyl group, 12-methylhenicosyl group, 13-methylhenicosyl group, 14-methylhenicosyl group, 15-methylhenicosyl group, 16-methylhenicosyl group, 17-methylhenicosyl group, 18-methylhenicosyl group, 19-methylhenicosyl group, 20-methylhenicosyl group; and the like.

When R¹ is a C₂₋₂₂ alkenyl group, examples of the alkenyl group include groups in which 1 to 3 of the saturated bonds between carbon molecules in the groups listed as the C₂₋₂₂ alkyl group are replaced by unsaturated bonds.

In the general formula (I), three R¹ groups in one molecule may be the same as or different from each other. In the ionic neutral lipid according to the present invention, from the viewpoint of ease of synthesis and quality stability, it is preferable that all three R¹ groups in one molecule are the same.

In the ionic neutral lipid according to the present invention, R¹ is preferably a linear C₁₋₂₂ alkyl group or a linear C₂₋₂₂ alkenyl group, and more preferably a linear C₁₅₋₂₂ alkyl group or a linear C₁₅₋₂₂ alkenyl group. In the ionic neutral lipid according to the present invention, it is more preferable that the three R¹ groups in one molecule are each the same group or different group selected from the group consisting of n-pentadecyl group, n-hexadecyl group, n-heptadecyl group, n-octadecyl group, n-nonadecyl group, n-icosyl group, n-henicosyl group, n-docosyl group, n-pentadecenyl group, n-hexadecenyl group, n-heptadecenyl group, n-octadecenyl group, n-nonadecenyl group, n-icosenyl group, n-henicosenyl group, and n-docosenyl group, and it is still more preferable that all three R¹ groups in one molecule are each the same group selected from the group consisting of n-pentadecyl group, n-hexadecyl group, n-heptadecyl group, n-octadecyl group, n-nonadecyl group, n-icosyl group, n-henicosyl group, n-docosyl group, n-pentadecenyl group, n-hexadecenyl group, n-heptadecenyl group, n-octadecenyl group, n-nonadecenyl group, n-icosenyl group, n-henicosenyl group, and n-docosenyl group.

In the general formula (I), a1 and a2 are each independently an integer of 0 to 4. When a1 is 0, -(CH₂)a1- means a single bond. Similarly, when a2 is 0, -(CH₂)a2-means a single bond. In the ionic neutral lipid according to the present invention, a1 is preferably an integer of 0 to 3. In the ionic neutral lipid according to the present invention, a2 is preferably an integer of 0 to 3, and more preferably an integer of 0 to 2. In the ionic neutral lipid according to the present invention, a1+a2 is preferably an integer of 1 to 6, and more preferably an integer of 1 to 4.

In the general formula (I), b1 and b2 are 0 or 1 satisfying b1+b2=1. When b1 is 1, b2 is 0, and when b1 is 0, b2 is 1. That is, the ionic neutral lipid represented by the general formula (I) necessarily has either one group of -NH₃⁺ or -(N⁺(R²)(R³))- in one molecule. Hereinafter, -NH₃⁺ and -(N⁺(R²)(R³))- may be collectively referred to as "ammonium cationic moiety."

In the general formula (I), R² and R³ are each independently a hydrogen atom or a C₁₋₃ alkyl group. Examples of the C₁₋₃ alkyl group include a methyl group, an ethyl group, an n-propyl group, and an isopropyl group. In the ionic neutral lipid according to the present invention, R² and R³ are preferably each independently a hydrogen atom, a methyl group, or an ethyl group, more preferably R² and R³ are each independently a hydrogen atom or a methyl group, and still more preferably R² is a hydrogen atom or a methyl group and R³ is a methyl group.

In the general formula (I), R⁴ is an anionic group. Examples of the anionic group include a carboxylic acid group (-C(=O)O⁻), a sulfonic acid group (-S(=O)₂O⁻), a phosphate group (-O-P(=O)O₂⁻), and a sulfate ester group (-OS(=O)O₂⁻). In the ionic neutral lipid according to the present invention, R⁴ is preferably a carboxylic acid group.

Examples of the ionic neutral lipid according to the present invention include compounds represented by the following general formulas (Z-9) to (Z-15). In the general formulas (Z-9) to (Z-15), R¹ is the same as in the general formula (I).

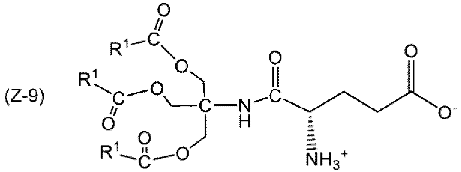

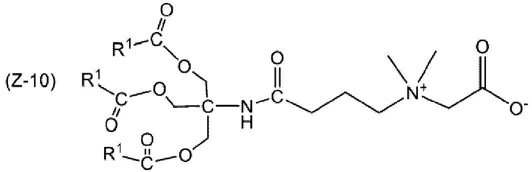

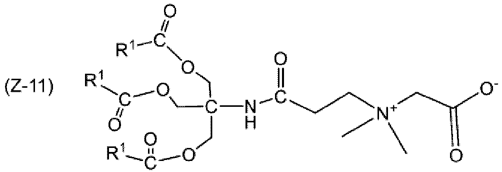

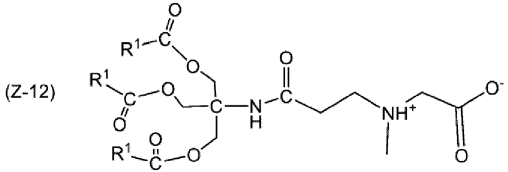

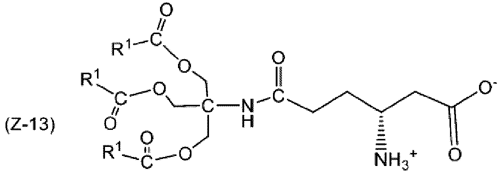

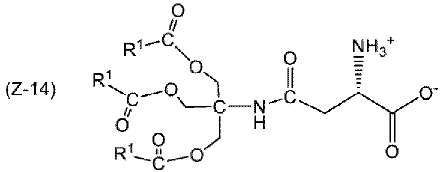

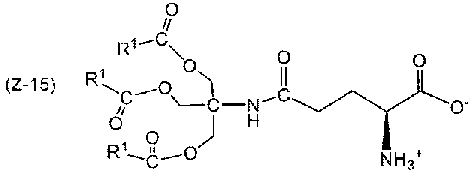

The ionic neutral lipid according to the present invention reduces the stability and makes it easier to undergo phase transition in a lipid membrane containing phosphatidylcholine. Therefore, by using the ionic neutral lipid according to the present invention as a constituent lipid of lipid nanoparticles, the uptake efficiency of the lipid nanoparticles into a cell membrane or the like containing phosphatidylcholine can be improved. The reason why the ionic neutral lipid according to the present invention has such an effect of suppressing the inhibition of phase transition of phosphatidylcholine is not clear, but it is presumed as follows.

In the ionic neutral lipid according to the present invention, three hydrocarbon chains composed of R¹ extend from one sp3 carbon, and a hydrophilic group having an anionic group at the terminal and an ammonium cationic moiety in front is further linked to the sp3 carbon. On the other hand, phosphatidylcholine has two hydrocarbon chains and a phosphocholine group, the phosphocholine group having a trimethylammonium group at the terminal and an anionic phosphate group moiety in front. Therefore, the ammonium cationic moiety and the anionic group in the hydrophilic group of the ionic neutral lipid according to the present invention can each form an ion pair with the phosphate group moiety and the trimethylammonium group in the phosphocholine group of phosphatidylcholine. The hydrophilic group of the ionic neutral lipid according to the present invention forms an ion pair with the phosphocholine group of phosphatidylcholine in the endosomal membrane, thereby changing the apparent shape of phosphatidylcholine to a cone shape and promoting the transition to a non-lamellar phase with negative curvature such as the inverse hexagonal phase. This phase transition to the non-lamellar phase promotes membrane fusion between the lipid nanoparticles and the endosomal membrane, which in turn promotes the uptake of the lipid nanoparticles into cells.

The ionic neutral lipid according to the present invention can be produced, for example, by condensing a compound represented by the following general formula (A) (hereinafter sometimes referred to as "compound (A)"), a compound represented by the following general formula (B) (hereinafter sometimes referred to as "compound (B)"), and tris(hydroxymethyl)aminomethane.

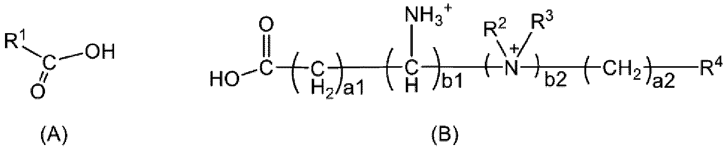

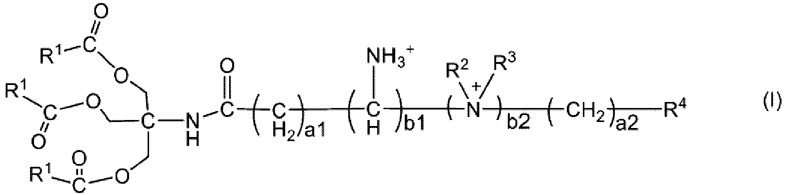

The ionic neutral lipid represented by the general formula (I) is synthesized by subjecting the carboxylic acid group of compound (A) and the hydroxyl group of Tris to dehydration condensation to form an ester bond, and further subjecting the carboxylic acid group of compound (B) and the amino group of Tris to dehydration condensation to form an amide bond,. The dehydration condensation reaction of the carboxylic acid group of compound (A) with the hydroxyl group of Tris can be generally carried out under the same reaction conditions as the dehydration condensation reaction in forming an ester of a carboxylic acid and an alcohol. The dehydration condensation reaction of the carboxylic acid group of compound (B) with the amino group of Tris can be generally carried out under the same reaction conditions as the dehydration condensation reaction in forming an amide of a carboxylic acid and an amine. That is, the ionic neutral lipid represented by the general formula (I) can be relatively easily synthesized by a general dehydration condensation reaction using Tris as a linker.

The ionic neutral lipid represented by the general formula (I) can be synthesized by reacting Tris with compound (A) to carry out a dehydration condensation reaction with the hydroxyl group of Tris, and then reacting the resulting ester with compound (B) to carry out a dehydration condensation reaction with the Tris-derived amino group. The ionic neutral lipid represented by the general formula (I) can also be synthesized by reacting Tris with compound (B) to carry out a dehydration condensation reaction with the amino group of Tris, and then reacting the resulting amide with compound (A) to carry out a dehydration condensation reaction with the Tris-derived hydroxyl group.

The ionic neutral lipid represented by the general formula (I) can be easily produced, for example, by the method specifically shown in the Examples of the present specification. By referring to this production method and appropriately selecting starting compounds, reagents, reaction conditions, and the like, those skilled in the art can easily produce any lipid included in the scope of the general formula (I).

### [Lipid Nanoparticle]

The lipid nanoparticle according to the present invention comprises the ionic neutral lipid according to the present invention as a constituent lipid. The lipid nanoparticle according to the present invention may comprise only one kind or two or more kinds of the ionic neutral lipid according to the present invention. When the lipid nanoparticle according to the present invention comprises two or more kinds of the ionic neutral lipid according to the present invention, the amount of the ionic neutral lipid according to the present invention means the total amount of lipid molecules corresponding to the ionic neutral lipid according to the present invention among the lipid molecules constituting the lipid nanoparticle.

The proportion of the ionic neutral lipid according to the present invention in the lipid molecules constituting the lipid nanoparticle is not particularly limited. For example, in the lipid nanoparticle according to the present invention, the ratio of the amount of the ionic neutral lipid according to the present invention to the total lipid amount constituting the lipid nanoparticle ([amount of ionic neutral lipid according to the present invention (mol)]/([total amount of lipid constituting the lipid nanoparticle (mol)])×100%) is preferably 1 mol% or more, more preferably 3 mol% or more, and further preferably 5 mol% or more. On the other hand, if the proportion of the ionic neutral lipid in the lipid molecules constituting the lipid nanoparticle is too high, the content of other lipids for imparting desired properties to the lipid nanoparticle may be too low. Therefore, the ratio of the amount of the ionic neutral lipid according to the present invention to the total lipid amount constituting the lipid nanoparticle in the lipid nanoparticle according to the present invention is preferably 90 mol% or less, more preferably 80 mol% or less, and further preferably 70 mol% or less. In the case of lipid nanoparticles used as a carrier for delivery to target cells in vivo, the ratio of the amount of the ionic neutral lipid according to the present invention to the total lipid amount constituting the lipid nanoparticle in the lipid nanoparticle according to the present invention is preferably 50 mol% or less, more preferably 30 mol% or less, and further preferably 5 to 20 mol%, since a higher delivery efficiency can be easily obtained.

As constituent lipids of the lipid nanoparticle according to the present invention, it is preferable to contain a pH-sensitive cationic lipid in addition to the ionic neutral lipid according to the present invention. The pH-sensitive cationic lipid is not particularly limited, and known pH-sensitive cationic lipids and modified compounds thereof can be appropriately used. Examples of known pH-sensitive cationic lipids include pH-sensitive cationic lipids described in Non-patent Literature 1 such as DLin-MC3-DMA, pH-sensitive cationic lipids described in Non-patent Literatures 2 to 4 such as YSK05 and YSK13-C3, and pH-sensitive cationic lipids described in Patent Literature 1 such as CL4H6. The pH-sensitive cationic lipid serving as a constituent lipid of the lipid nanoparticle according to the present invention together with the ionic neutral lipid according to the present invention has preferably a pKa of 6.6 or less, more preferably a pKa of 5.8 to 6.6, and further preferably a pKa of 6.0 to 6.6.

As the lipid other than the ionic neutral lipid according to the present invention and the pH-sensitive cationic lipid among the constituent lipids of the lipid nanoparticle according to the present invention, lipids generally used in forming liposomes can be used. Examples of such lipids include phospholipids, sterols, glycolipids, and saturated or unsaturated fatty acids. These can be used alone or in combination of two or more.

Examples of phospholipids include glycerophospholipids such as phosphatidylserine, phosphatidylinositol, phosphatidylglycerol, phosphatidylethanolamine, phosphatidylcholine, cardiolipin, plasmalogen, ceramide phosphorylglycerol phosphate, and phosphatidic acid; and sphingophospholipids such as sphingomyelin, ceramide phosphorylglycerol, and ceramide phosphorylethanolamine. Naturally occurring phospholipids such as egg yolk lecithin and soybean lecithin can also be used. The fatty acid residues in glycerophospholipids and sphingophospholipids are not particularly limited, but examples thereof include saturated or unsaturated fatty acid residues having 12 to 24 carbon atoms, and saturated or unsaturated fatty acid residues having 14 to 20 carbon atoms are preferable. Specific examples include acyl groups derived from fatty acids such as lauric acid, myristic acid, palmitic acid, palmitoleic acid, stearic acid, oleic acid, linoleic acid, linolenic acid, arachidic acid, arachidonic acid, behenic acid, and lignoceric acid. When these glycerolipids or sphingolipids have two or more fatty acid residues, all the fatty acid residues may be the same group or different groups.

Examples of sterols include animal-derived sterols such as cholesterol, cholesterol hemisuccinate, lanosterol, dihydrolanosterol, desmosterol, and dihydrocholesterol; plant-derived sterols (phytosterols) such as stigmasterol, sitosterol, campesterol, and brassicasterol; and microorganism-derived sterols such as timosterol and ergosterol. Examples of glycolipids include glycosylglycerols such as sulfoxyribosyl glyceride, diglycosyl diglyceride, digalactosyl diglyceride, galactosyl diglyceride, and glycosyl diglyceride; and glycosphingolipids such as galactosylceramide, lactosylceramide, and gangliosides. Examples of saturated or unsaturated fatty acids include saturated or unsaturated fatty acids having 12 to 20 carbon atoms such as palmitic acid, oleic acid, stearic acid, arachidonic acid, and myristic acid.

It is preferable that the constituent lipids of the lipid nanoparticle according to the present invention include another neutral lipid in addition to the ionic neutral lipid according to the present invention, more preferably a phospholipid or a sterol, further preferably a sterol, and still more preferably cholesterol.

The lipid nanoparticle according to the present invention preferably contains a polyalkylene glycol-modified lipid as a lipid component. Since polyalkylene glycol is a hydrophilic polymer, the surface of a lipid nanoparticle can be modified with polyalkylene glycol by constructing the lipid nanoparticle using a polyalkylene glycol-modified lipid as a constituent lipid of a lipid membrane. Surface modification with polyalkylene glycol may enhance the stability of the lipid nanoparticle such as the blood retention property.

As the polyalkylene glycol, for example, polyethylene glycol, polypropylene glycol, polytetramethylene glycol, polyhexamethylene glycol, or the like can be used. The molecular weight of the polyalkylene glycol is, for example, about 300 to about 10,000, preferably about 500 to about 10,000, and further preferably about 1,000 to about 5,000.

For example, stearylated polyethylene glycol (e.g., stearic acid PEG45 (STR-PEG45)) can be used for modification of a lipid with polyethylene glycol. In addition, polyethylene glycol derivatives such as N-[carbonyl-methoxypolyethylene glycol-2000]-1,2-dipalmitoyl-sn-glycero-3-phosphoethanolamine, N-[carbonyl-methoxypolyethylene glycol-5000]-1,2-dipalmitoyl-sn-glycero-3-phosphoethanolamine, N-[carbonyl-methoxypolyethylene glycol-750]-1,2-distearoyl-sn-glycero-3-phosphoethanolamine, N-[carbonyl-methoxypolyethylene glycol-2000]-1,2-distearoyl-sn-glycero-3-phosphoethanolamine, N-[carbonyl-methoxypolyethylene glycol-5000]-1,2-distearoyl-sn-glycero-3-phosphoethanolamine, and 1,2-dimyristoyl-rac-glycero-3-methoxypolyethylene glycol-2000 (PEG-DMG) can also be used, but polyalkylene glycolated lipids are not limited thereto.

The proportion of the polyalkylene glycol-modified lipid to the total lipid amount constituting the lipid nanoparticle according to the present invention is not particularly limited as long as it does not impair the effect of suppressing the inhibition of phase transition of phosphatidylcholine by the ionic neutral lipid according to the present invention. For example, the proportion of the polyalkylene glycol-modified lipid to the total lipid amount constituting the lipid nanoparticle is preferably 0.5 to 3 mol%.

The lipid nanoparticle according to the present invention can be subjected to appropriate surface modification or the like as necessary.

The blood retention property of the lipid nanoparticle according to the present invention can be enhanced by modifying the surface with a hydrophilic polymer or the like. Surface modification may be performed by using a lipid modified with these modifying groups as a constituent lipid of the lipid nanoparticle.

In producing the lipid nanoparticle according to the present invention, for example, glycophorin, ganglioside GM1, phosphatidylinositol, ganglioside GM3, glucuronic acid derivative, glutamic acid derivative, polyglycerin phospholipid derivative, or the like can also be used as a lipid derivative for enhancing the blood retention property. In addition to polyalkylene glycol, dextran, pullulan, Ficoll, polyvinyl alcohol, styrene-maleic anhydride alternating copolymer, divinyl ether-maleic anhydride alternating copolymer, amylose, amylopectin, chitosan, mannan, cyclodextrin, pectin, carrageenan, or the like can also be used for surface modification as the hydrophilic polymer for enhancing the blood retention property.

Further, in order to promote nuclear translocation of the lipid nanoparticle according to the present invention, for example, the surface of the lipid nanoparticle can be modified with an oligosaccharide compound having three or more saccharides. The type of the oligosaccharide compound having three or more saccharides is not particularly limited, but for example, an oligosaccharide compound in which about 3 to about 10 sugar units are bonded can be used, and preferably an oligosaccharide compound in which about 3 to about 6 sugar units are bonded can be used. Among them, preferably, an oligosaccharide compound which is a trimer to hexamer of glucose can be used, and more preferably, an oligosaccharide compound which is a trimer or tetramer of glucose can be used. More specifically, isomaltotriose, isopanose, maltotriose, maltotetraose, maltopentaose, or maltohexaose can be preferably used, and among them, maltotriose, maltotetraose, maltopentaose, or maltohexaose in which glucose is α1-4 bonded is more preferable. Particularly preferable is maltotriose or maltotetraose, and most preferable is maltotriose. The amount of the surface modification of the lipid nanoparticle with the oligosaccharide compound is not particularly limited, but is, for example, about 1 to about 30 mol%, preferably about 2 to about 20 mol%, and more preferably about 5 to about 10 mol% with respect to the total lipid amount.

The method for modifying the surface of the lipid nanoparticle with an oligosaccharide compound is not particularly limited; for example, a liposome in which the surface of the lipid nanoparticle is modified with a monosaccharide such as galactose or mannose (International Publication No. WO 2007/102481) is known, and thus the surface modification method described in this publication can be employed. All of the disclosure of the above-mentioned publication is included in the disclosure of this specification by reference.

Further, the lipid nanoparticle according to the present invention can be imparted with, for example, any one or two or more functions such as a temperature change-sensitive function, a membrane permeation function, a gene expression function, and a pH-sensitive function. By appropriately adding these functions, it is possible to improve the retention property of the lipid nanoparticle in blood, efficiently release the lipid nanoparticle from the endosome after endocytosis in the target cell, and more efficiently express the encapsulated nucleic acid in the target cell.

The lipid nanoparticle according to the present invention may contain one or more substances selected from the group consisting of antioxidants such as tocopherol, propyl gallate, ascorbyl palmitate, or butylated hydroxytoluene, charged substances, and membrane polypeptides. Examples of the charged substances for imparting a positive charge include saturated or unsaturated aliphatic amines such as stearylamine and oleylamine, and examples of the charged substances for imparting a negative charge include dicetyl phosphate, cholesteryl hemisuccinate, phosphatidylserine, phosphatidylinositol, and phosphatidic acid. Examples of membrane polypeptides include peripheral membrane polypeptides and integral membrane polypeptides. The blending amount of these substances is not particularly limited and can be appropriately selected depending on the purpose.

The size of the lipid nanoparticle according to the present invention is preferably an average particle size of 400 nm or less, more preferably an average particle size of 300 nm or less, further preferably an average particle size of 200 nm or less, and still more preferably 150 nm or less, since a high delivery efficiency to target cells in vivo can be easily obtained. The average particle size of the lipid nanoparticle means a number average particle size measured by dynamic light scattering (DLS). Measurement by dynamic light scattering can be carried out by a conventional method using a commercially available DLS apparatus or the like.

The polydispersity index (PDI) of the lipid nanoparticle according to the present invention is about 0.01 to about 0.7, preferably about 0.01 to about 0.6, and further preferably about 0.01 to about 0.3. The zeta potential can be in the range of -50 mV to 5 mV, preferably in the range of -10 mV to 5 mV.

The form of the lipid nanoparticle according to the present invention is not particularly limited, and examples thereof include a unilamellar liposome, a multilamellar liposome, a spherical micelle, and an amorphous layered structure, as a form dispersed in an aqueous solvent. The lipid nanoparticle according to the present invention is preferably a unilamellar liposome or a multilamellar liposome.

The lipid nanoparticle according to the present invention preferably encapsulates a component of interest to be delivered into a target cell inside the particle covered with a lipid membrane. The component encapsulated inside the particle by the lipid nanoparticle according to the present invention is not particularly limited as long as it has a size that can be encapsulated. Any substance such as a nucleic acid, a saccharide, a peptide, a low molecular weight compound, and a metal compound can be encapsulated in the lipid nanoparticle according to the present invention.

A nucleic acid is preferable as the component to be encapsulated in the lipid nanoparticle according to the present invention. The nucleic acid may be DNA, RNA, or an analog or derivative thereof (e.g., peptide nucleic acid (PNA) or phosphorothioate DNA). The nucleic acid to be encapsulated in the lipid nanoparticle according to the present invention may be a single-stranded nucleic acid or a double-stranded nucleic acid, and may be linear or circular.

The nucleic acid to be encapsulated in the lipid nanoparticle according to the present invention preferably contains a foreign gene to be expressed in a target cell, and more preferably is a nucleic acid that functions to express the foreign gene in a cell by being taken up into the cell. The foreign gene may be a gene originally contained in the genomic DNA of the target cell or a gene not contained in the genomic DNA. Examples of such nucleic acids include gene expression vectors containing a nucleic acid comprising a base sequence encoding a gene to be expressed. The gene expression vector may exist as an extrachromosomal gene in a transfected cell, or may be integrated into genomic DNA by homologous recombination.

The gene expression vector to be encapsulated in the lipid nanoparticle according to the present invention is not particularly limited, and a vector generally used in gene therapy or the like can be used. The gene expression vector to be encapsulated in the lipid nanoparticle according to the present invention is preferably a nucleic acid vector such as a plasmid vector. The plasmid vector may be in a circular form, or may be encapsulated in the lipid nanoparticle according to the present invention in a state of being linearly cleaved in advance. The gene expression vector can be designed by a conventional method with generally used molecular biological tools based on the base sequence information of the target gene to be expressed, and can be produced by various known methods.

The nucleic acid to be encapsulated in the lipid nanoparticle according to the present invention is also preferably a functional nucleic acid that controls the expression of a target gene present in a target cell. Examples of the functional nucleic acid include antisense oligonucleotides, antisense DNA, antisense RNA, siRNA, microRNA, and mRNA. It may also be plasmid DNA (pDNA) serving as an siRNA expression vector that expresses siRNA in a cell. The siRNA expression vector can be prepared from a commercially available siRNA expression vector, or can be appropriately modified. The nucleic acid to be encapsulated in the lipid nanoparticle according to the present invention is preferably mRNA or pDNA because of its particularly good selectivity for tissues in vivo.

The method for producing the lipid nanoparticle according to the present invention is not particularly limited, and any method available to those skilled in the art can be employed. It can be produced as follows: for example, all lipid components are dissolved in an organic solvent such as chloroform, and a lipid membrane is formed by drying under reduced pressure using an evaporator or spray drying using a spray dryer, and then a component to be encapsulated in the lipid nanoparticle, for example, an aqueous solvent containing a nucleic acid or the like, is added to the dried mixture, and further emulsified by an emulsifier such as a homogenizer, an ultrasonic emulsifier, or a high-pressure jet emulsifier. It can also be produced by a method well known as a method for producing liposomes, such as a reverse phase evaporation method. When it is desired to control the size of the lipid nanoparticle, extrusion through a membrane filter having a uniform pore size or the like under high pressure may be performed.

The composition of the aqueous solvent (dispersion medium) is not particularly limited, but examples thereof include buffer solutions such as phosphate buffer, citrate buffer, and phosphate buffered saline, physiological saline, and cell culture media. These aqueous solvents (dispersion media) can stably disperse the lipid nanoparticles, but further, sugars (aqueous solutions) such as monosaccharides such as glucose, galactose, mannose, fructose, inositol, ribose, and xylose, disaccharides such as lactose, sucrose, cellobiose, trehalose, and maltose, trisaccharides such as raffinose and melezitose, polysaccharides such as cyclodextrin, and sugar alcohols such as erythritol, xylitol, sorbitol, mannitol, and maltitol, and polyhydric alcohols (aqueous solutions) such as glycerin, diglycerin, polyglycerin, propylene glycol, polypropylene glycol, ethylene glycol, diethylene glycol, triethylene glycol, polyethylene glycol, ethylene glycol monoalkyl ether, diethylene glycol monoalkyl ether, and 1,3-butylene glycol may be added. In order to stably store the lipid nanoparticles dispersed in this aqueous solvent for a long period of time, it is desirable to exclude electrolytes in the aqueous solvent as much as possible from the viewpoint of physical stability such as aggregation suppression. From the viewpoint of chemical stability of the lipid, it is desirable to set the pH of the aqueous solvent to near neutral from weakly acidic (about pH 3.0 to 8.0) and/or to remove dissolved oxygen by nitrogen bubbling or the like.

The lipid nanoparticle according to the present invention can also be produced by an alcohol dilution method using a flow path. The method is a method for producing lipid nanoparticles by introducing a solution in which lipid components are dissolved in an alcohol solvent and a solution in which a water-soluble component to be included in the lipid nanoparticle is dissolved in an aqueous solvent from separate flow paths and merging them. By using a microchannel with a built-in three-dimensional micromixer capable of achieving instantaneous mixing of two liquids, lipid nanoparticles having a diameter of about 30 nm can be reproducibly produced (Non-patent Literature 6). As the flow path used for production, it is preferable to use a simple two-dimensional flow path structure in which baffles of a certain width are arranged alternately from both sides in a micro-sized flow path through which a raw material solution flows, as described in Patent Literature 2, since it can form a nano-sized lipid particle formation system with high particle size controllability. As the aqueous solvent used in the alcohol dilution method, the solvents described above can be used.

When the resulting aqueous dispersion of the lipid nanoparticle is freeze-dried or spray-dried, for example, a use of sugars (aqueous solutions) such as monosaccharides such as glucose, galactose, mannose, fructose, inositol, ribose, and xylose, disaccharides such as lactose, sucrose, cellobiose, trehalose, and maltose, trisaccharides such as raffinose and melezitose, polysaccharides such as cyclodextrin, and sugar alcohols such as erythritol, xylitol, sorbitol, mannitol, and maltitol may improve stability. Further, when the aqueous dispersion is frozen, for example, a use of the above-mentioned sugars or polyhydric alcohols (aqueous solutions) such as glycerin, diglycerin, polyglycerin, propylene glycol, polypropylene glycol, ethylene glycol, diethylene glycol, triethylene glycol, polyethylene glycol, ethylene glycol monoalkyl ether, diethylene glycol monoalkyl ether, and 1,3-butylene glycol may improve stability.

The lipid nanoparticle according to the present invention functions as a gene expression carrier to a target tissue. By encapsulating a foreign gene to be expressed in a target cell in the lipid nanoparticle according to the present invention and administering the lipid nanoparticle to a subject animal, the foreign gene is expressed in the target tissue of the subject animal. Therefore, the lipid nanoparticle according to the present invention is useful as an active ingredient of a pharmaceutical composition used for gene therapy.

The animal to which the lipid nanoparticle according to the present invention is administered is not particularly limited, and may be a human or an animal other than a human. Examples of non-human animals include mammals such as cattle, pigs, horses, sheep, goats, monkeys, dogs, cats, rabbits, mice, rats, hamsters, and guinea pigs, and birds such as chickens, quails, and ducks. The administration route for administering the lipid nanoparticle according to the present invention to an animal is not particularly limited, but is preferably parenteral administration such as intravenous administration, enteral administration, intramuscular administration, subcutaneous administration, transdermal administration, nasal administration, and pulmonary administration.

### Examples

Next, the present invention will be described in more detail with reference to Examples, but the present invention is not limited to the following Examples.

### <NMR (Nuclear Magnetic Resonance)>

In the following experiments, unless otherwise specified, ¹H and ¹³C NMR spectra were obtained using an NMR apparatus manufactured by JEOL Ltd. (ECA500 apparatus, ECZ400 apparatus, or ECX400P apparatus) with tetramethylsilane as an internal standard (0 ppm), wherein d is an abbreviation for doublet, t is an abbreviation for triplet, and m is an abbreviation for multiplet. Chemical shifts of the ¹H NMR spectra were reported in ppm on the σ scale, downfield from tetramethylsilane.

### <Constituent Lipids of Lipid Nanoparticles>

7-(4-(Diisopropylamino)butyl)-7-hydroxytridecane-1,13-diyl dioleate (CL4H6) and 7-hydroxy-7-(4-((1-methylpiperidine-4-carbonyl)oxy)butyl)tridecane-1,13-diyl dioleate (CL15H6) synthesized by the methods described in Patent Literature 1 were used. 7-(4-(Diisopropylamino)butyl)-13-((2-hexylnonanoyl)oxy)-7-hydroxytridecyl 2-hexyldecanoate (CL4F6) synthesized in Synthesis Example 1 below was used. 7-Hydroxy-7-(4-((1-methylpiperidine)-4-carbonyl)oxy)butyl)tridecane-1,13-diyl bis(2-hexyldecanoate) (CL15F6) synthesized by the methods described in International Publication No. WO 2022/071582 was used. ALC-0315 manufactured by Ambeed, DLin-MC3-DMA (MC3) manufactured by Selleck Biotech, SM-102 manufactured by Cayman Chemical, and cholesterol (Chol) manufactured by SIGMA Aldrich were respectively used. 1,2-Distearoyl-sn-glycero-3-phosphocholine (DSPC), 1,2-dioleoyl-sn-glycero-3-phosphocholine (DOPC), 1,2-dioleoyl-sn-glycero-3-phosphoethanolamine (DOPE), and methoxyethylene glycol 2000-modified 2-dimyristoyl-rac-glycerol (PEG-DMG) manufactured by NOF CORPORATION were used.

### <Evaluation of Lipid Polymorphism by ³¹P NMR Spectroscopy>

The spectrum of a lipid membrane by ³¹P NMR spectroscopy differs depending on the phase. Therefore, analysis of a lipid membrane composed of DOPC and a lipid under evaluation was performed by ³¹P NMR spectroscopy, the polymorphism of the phase state of the lipid membrane was evaluated from the obtained spectrum, and the ability of the lipid under evaluation to destroy the DOPC membrane was evaluated.

Specifically, DOPC and a lipid under evaluation (DOPE or TOT-10) were mixed at a molar ratio of 1:1 (16 µmol of total lipid) and were dissolved in 300 µL of chloroform, and then the solvent was removed by a distillation apparatus (rotovap) to form a lipid membrane. The resulting lipid membrane was hydrated with 500 µL of PBS(-) at 60°C to obtain a sample for measurement.

Next, the sample was transferred to an NMR tube, and a proton-decoupled ³¹P NMR spectrum was obtained using an ECA500 apparatus manufactured by JEOL Ltd. Acquisition parameters included a 60° pulse, a spectral width of 280 kHz (for ECX 400P) with 32,768 data points, and an interpulse delay time of 1 second. The temperature was controlled at 25°C. Prior to Fourier transform, exponential multiplication corresponding to a line broadening of 50 Hz was applied to the free induction decay. Chemical shifts were referenced to 85% phosphoric acid (H₃PO₄) as an external standard.

### <Thin Layer Chromatography (TLC)>

In the following experiments, unless otherwise specified, the reactants of all reactions were monitored by TLC using pre-coated TLC plates (manufactured by Millipore). TLC visualization was performed by UV light (254 nm), phosphomolybdic acid staining, or p-anisaldehyde staining. Reaction products were purified by an automated CombiFlash (registered trademark) Rf Chromatography System (manufactured by Teledyne ISCO) or a Selekt system (manufactured by Biotage).

### <mRNA>

For the nucleic acids to be encapsulated in lipid nanoparticles, mRNA encoding firefly luciferase (Fluc) manufactured by TriLink Biotechnologies (CleanCap FLuc mRNA, 5moU) was used.

### <Preparation of Lipid Nanoparticles>

In the following experiments, unless otherwise specified, lipid nanoparticles were prepared by an alcohol dilution method using a flow path. As the flow path, a micromixer-integrated microfluidic device "iLiNP" (manufactured by Lilac Pharma) was used. The flow rate of the device was controlled using a syringe pump (Harvard Apparatus).

Specifically, first, 80 µL of an ethanol solution containing each lipid component at a predetermined molar ratio and adjusted to a total lipid concentration of 8 mM and 240 µL of an acetate buffer (25 mM, pH 4.0) adjusted to a nucleic acid concentration of 55 µg/mL were fed into a microchannel, and a lipid nanoparticle solution discharged from the flow path was collected. The lipid nanoparticle solution was placed in a dialysis membrane (MWCO 12,000-14,000, manufactured by Spectrum Laboratories), dialyzed against PBS(-) (pH 7.4) as an external aqueous phase at 4°C for 2 hours or more, and then the lipid nanoparticle solution was collected from the dialysis membrane.

### <Measurement of Average Particle Size and Zeta Potential of Lipid Nanoparticles>

The average particle size (number average value), ζ-average particle size, and polydispersity index (PdI) of the lipid nanoparticles in PBS(-) (pH 7.4), and the zeta potential in 10 mM HEPES buffer (pH 7.4) were measured using an analyzer "Zetasizer Nano ZS ZEN3600" (manufactured by Malvern) utilizing dynamic light scattering.

### <Encapsulation Rate (Encapsulation Efficiency) of Lipid Nanoparticles>

The encapsulation rate of the lipid nanoparticles was determined by measuring the amount of encapsulated nucleic acid (mRNA) using RiboGreen (manufactured by Life Technologies), which is an RNA intercalator.

### <Measurement of Apparent pKa of Lipid Nanoparticles>

The apparent pKa of the lipid nanoparticles was measured using p-toluenesulfonic acid (TNS). First, TNS (final concentration: 0.75 µM) and lipid nanoparticles (final concentration: 30 µM) were mixed in a buffer (200 mL) adjusted to each pH (pH 3.5 to 9.5). As the buffer, 20 mM citrate buffer, 20 mM sodium phosphate buffer, or 20 mM Tris-HCl buffer containing 130 mM NaCl was used. The fluorescence intensity of the prepared mixture was measured using a fluorescence spectrophotometer (Varioskan Lux, manufactured by Thermo Fisher Scientific) with settings of λex=321 nm and λem=447 nm. Among the measured values, the highest value and the lowest value were set as 100% and 0% ionization degrees, respectively, and the pH showing a 50% ionization degree was calculated as the apparent pKa. The content ratio (%) of cationically charged ionic neutral lipid was calculated using the Henderson-Hasselbalch equation.

### <Measurement of Fluc Activity>

First, BALB/c mice (4 weeks old, female) were administered with lipid nanoparticles encapsulating Fluc mRNA (Fluc mRNA-loaded lipid nanoparticles) by intravenous injection, intramuscular injection, or intracerebral injection. The mRNA doses (volumes) for intravenous injection, intramuscular injection, and intracerebral injection were 0.1 mg/10 mL/kg, 1 µg/50 µL/mouse, and 0.1 µg/5 µL/mouse, respectively. Six hours after administration, the mice were euthanized, and each tissue (liver, quadriceps femoris muscle, brain) was collected, frozen in liquid nitrogen, and stored at -80°C. The tissues were homogenized in passive lysis buffer (manufactured by Promega) using a bead-type cell disruptor (Micro Smash MS-100R, manufactured by TOMY Seiko). Tissue debris was removed by centrifugation, and the supernatant was collected. Fluc activity in the supernatant was measured using the Luciferase Assay System (E1500, manufactured by Promega) according to the manufacturer's protocol. Luminescence was measured using a luminometer (Luminescencer-PSN, manufactured by ATTO). Protein concentration was determined using a commercially available assay kit (BCA Protein Assay Kit, manufactured by Pierce). Fluc activity was expressed as relative light units (RLU) per mg of protein.

### <Hemolysis Test>

In the hemolysis test, lipid nanoparticles were added to erythrocytes, and hemolysis caused by erythrocyte membrane disruption was measured. By using a low pH buffer to reproduce the pH inside the endosome, the membrane fusion ability of the lipid nanoparticles was measured and evaluated as the endosomal escape ability. Specifically, blood conditions, early endosomes, and late endosomes were evaluated using simulated conditions with a pH 7.4 buffer, a pH 6.0 buffer, and a pH 5.0 buffer, respectively.

10 mM malate buffer (120 mM NaCl) was used as a pH 5.0 buffer, 10 mM HEPES buffer (120 mM NaCl) was used as a pH 6.0 buffer, and 10 mM MES buffer (120 mM NaCl) was used as a pH 7.4 buffer.

To a transparent 96-well plate, a lipid nanoparticle solution having a total lipid concentration of 0.75 mM and a buffer of each pH were added, and the lipid nanoparticle solution was diluted with the buffer of each pH so that the final lipid molar amount was 0.781 nmol to 12.5 nmol. The total volume was 100 µL. As a positive control, 90 µL of a buffer of each pH was added to 10 µL of a physiological saline solution containing 10% Triton X-100 to make a total volume of 100 µL. As a negative control, 100 µL of a buffer of each pH was added.

To these lipid nanoparticle diluted solutions, 100 µL of a blood solution diluted with a buffer of the same pH was added to make a total volume of 200 µL per well, and shaken at 37°C and 900 rpm for 30 minutes. Thereafter, the plate was centrifuged at 4°C and 400×g for 5 minutes. 100 µL of the supernatant was taken from each well of the plate after centrifugation and added to each well of a transparent 96-well plate for absorbance measurement. 100 µL of physiological saline was added in advance to the wells of the positive control of the transparent 96-well plate for absorbance measurement, 100 µL of 0.5% Triton X-100 was added in advance to each well of the negative control and the lipid nanoparticle-added sample, and the final concentration of Triton X-100 in each well was adjusted to 0.25%. The absorbance at 545 nm was measured for the plate having a total volume of 200 µL per well using a fluorescence spectrophotometer (Varioskan Lux, manufactured by Thermo Fisher Scientific), and the hemolytic activity when the negative control was used as a blank and the positive control was used as 100% was calculated.

The blood solution was prepared from a fresh mouse erythrocyte suspension as follows.

First, the erythrocyte suspension was prepared by euthanizing BALB/c mice (4 weeks old, female), collecting blood from the inferior vena cava, and adding 1 mL of blood to a 1.5 mL tube containing 1 µL of 10 mg/mL heparin. 1 mL of the heparinized blood was transferred to a 50 mL tube and diluted by adding 9 mL of physiological saline. The diluted blood was centrifuged at 4°C and 400×g for 5 minutes, and then the supernatant was removed. After repeating this operation 5 times, 10 mL of physiological saline was added to obtain an erythrocyte suspension.

The erythrocyte concentration of the erythrocyte suspension was determined as follows. To a transparent 96-well plate, 10 µL of a physiological saline solution containing 10% Triton X-100 and erythrocyte suspension or physiological saline were added, and the erythrocyte suspension was diluted so that the total volume was 200 µL and the final concentration of Triton X-100 was 0.25%. The erythrocyte suspension was diluted to have a dilution factor of 6.6 to 200 times. The plate containing the solution was shaken at 37°C and 700 rpm for 1 minute, 100 µL of the solution after shaking was taken from each well, 100 µL of physiological saline was added, and 2-fold diluted. The absorbance at 545 nm was measured for the plate having a total volume of 200 µL per well using a fluorescence spectrophotometer (Varioskan Lux, manufactured by Thermo Fisher Scientific), and a calibration curve was drawn. From the calibration curve, the dilution factor of the erythrocyte suspension at which the absorbance becomes 1.0 when diluted 4 times was calculated. The erythrocyte suspension was diluted with a buffer of each pH so as to have the dilution factor to prepare a blood solution.

### [Synthesis Example 1] Synthesis of CL4F6

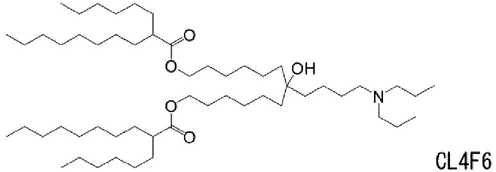

7-(4-(Diisopropylamino)butyl)tridecane-1,7,13-triol (1.0 mmol) synthesized by the method described in Patent Literature 1 was dissolved in 5 mL of dichloromethane, followed by an addition of 2-hexyldecanoic acid (2.20 mmol), DMAP (N,N-dimethyl-4-aminopyridine) (0.20 mmol), and EDCI (1-ethyl-3-(3-dimethylaminopropyl)carbodiimide) (3.0 mmol), and reacted at room temperature overnight. After removing the solvent using a rotary evaporator, the residue was suspended in ethyl acetate, and insoluble matters were removed by filtration. The filtrate was washed by liquid separation with 0.5 N aqueous sodium oxide solution and saturated brine. Anhydrous sodium sulfate was added to the organic layer for dehydration. After filtration, the solvent was removed using a rotary evaporator to obtain a crude product. The crude product was purified by silica gel chromatography [eluting solvent; dichloromethane:methanol (continuous gradient)] to obtain CL4F6.

### [Example 1]

An oleic acid residue was condensed to the hydroxyl group of Tris, and then to the Tris-derived amino group of the resulting ester, 5-tert-butyl N-((9H-fluoren-9-ylmethoxy)carbonyl)-D-glutamic acid hydrate was condensed to synthesize (S)-4-amino-5-((1,3-bis(oleoyloxy)-2-((oleoyloxy)methyl)propan-2-yl)amino)-5-oxopen tanoic acid (TOT-9).

### (1) Synthesis of tert-butyl (1,3-dihydroxy-2-(hydroxymethyl)propan-2-yl)carbamate (Compound 1)

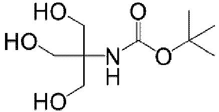

Di-tert-butyl dicarbonate (23.5 g, 107.7 mmol) dissolved in tert-butanol (75 mL) was added dropwise to a solution in which Tris (10.0 g, 82.6 mmol) was dispersed in a mixed solvent of methanol (75 mL) and tert-butanol (75 mL). The resulting reaction mixture was stirred at ambient temperature overnight and then evaporated. The resulting residue was purified by precipitation with cold EtOAc. Vacuum filtration gave compound 1 (16.7 g, yield 91%) as a white powder.

### (2) Synthesis of 2-((tert-butoxycarbonyl)amino)-2-((oleoyloxy)methyl)propane-1,3-diyl dioleate (Compound 2)

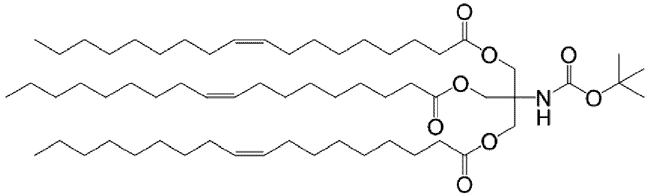

Compound 1 (2.21 g, 10.0 mmol) was dissolved in anhydrous DCM (dichloromethane, 50 mL), and further oleic acid (9.32 g, 33.0 mmol), DMAP (N,N-dimethyl-4-aminopyridine) (122 mg, 1.0 mmol), and EDCI-HCl (1-(3-dimethylaminopropyl)-3-ethylcarbodiimide hydrochloride) (7.67 g, 40.0 mmol) were dissolved in the resulting mixture. The resulting reaction mixture was stirred at ambient temperature overnight and then evaporated. The resulting residue was suspended with EtOAc, washed with saturated citric acid solution and water, and then dried over anhydrous Na₂SO₄. The solution was filtered, and the filtrate was evaporated. The residue was loaded on a normal-phase column (Sfär Silica HCD, manufactured by Biotage) and purified by flash chromatography with a gradient mobile phase of hexane and EtOAc. This gave compound 2 (8.50 g, yield 84%) as a colorless oil.

### (3) Synthesis of 22-amino-2-((oleoyloxy)methyl)propane-1,3-diyl dioleate (TOT-0)

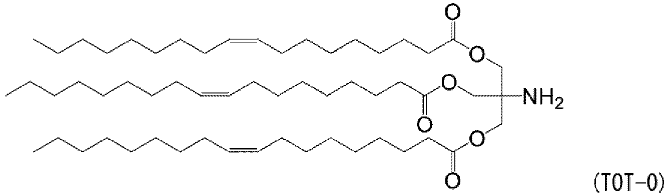

Compound 2 (8.50 g, 8.38 mmol) was dissolved in anhydrous DCM (44 mL), and TFA (trifluoroacetic acid) (5 mL) and TIPS (triisopropylsilane) (1 mL) were added to the resulting mixture. The resulting reaction mixture was stirred at ambient temperature for 3 hours. The reaction mixture was diluted with EtOAc, and the organic phase was washed with 1 N aqueous NaOH solution, water, and brine, and dried over anhydrous Na₂SO₄. The solution was filtered, and the filtrate was evaporated. The residue was loaded on a normal-phase column (Sfär Silica HCD, manufactured by Biotage) and purified by flash chromatography with a gradient mobile phase of hexane and EtOAc. This gave TOT-0 (5.00 g, yield 65%) as a colorless oil.

### (4) Synthesis of (S)-4-amino-5-((1,3-bis(oleoyloxy)-2-((oleoyloxy)methyl)propan-2-yl)amino)-5-oxopen tanoic acid (TOT-9)

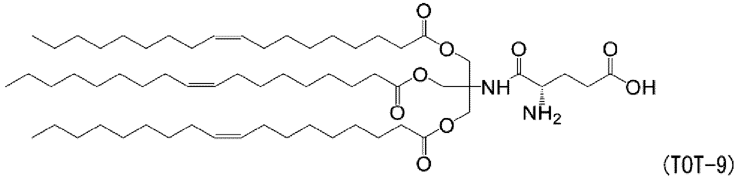

TOT-0 (366 mg, 0.40 mmol) was dissolved in ethanol (4 mL), and 5-tert-butyl N-((9H-fluoren-9-ylmethoxy)carbonyl)-D-glutamic acid hydrate (170 mg, 0.44 mmol) and DMT-MM (4-(4,6-dimethoxy-1,3,5-triazin-2-yl)-4-methylmorpholinium chloride) (122 mg, 0.44 mmol) were added to the resulting mixture. The reaction mixture was stirred at ambient temperature overnight and then evaporated. The resulting residue was suspended in EtOAc (4 mL), washed with 0.5 N NaOH aq. (5 mL), and then dried over anhydrous Na₂SO₄. The resulting solution was filtered, and the filtrate was evaporated. The resulting solution was dissolved in anhydrous DCM (2.8 mL), and TFA (0.8 mL) and TIPS (0.4 mL) were added to the resulting mixture. The reaction mixture was stirred at ambient temperature overnight and then diluted with EtOAc, and the resulting organic phase was washed with 1 N aqueous NaOH solution, water and brine, and dried over anhydrous Na₂SO₄. The resulting solution was filtered, and the filtrate was evaporated. DMF (1.8 mL) and piperidine (200 µL) were added to the resulting solution, followed by stirring at ambient temperature overnight and then evaporation. The resulting residue was loaded on a normal-phase column (Sfär Silica HCD, manufactured by Biotage) and purified by flash chromatography with a gradient mobile phase of hexane and EtOAc. This gave TOT-9 (213 mg, yield 51%) as a colorless viscous oil.

### [Example 2]

4-(Dimethylamino)butyric acid was condensed to the amino group in TOT-0 synthesized in Example 1 to synthesize 2-((4-((1,3-bis(oleoyloxy)-2-((oleoyloxy)methyl)propan-2-yl)amino)-4-oxobutyl)dimeth ylammonio)acetate (TOT-10).

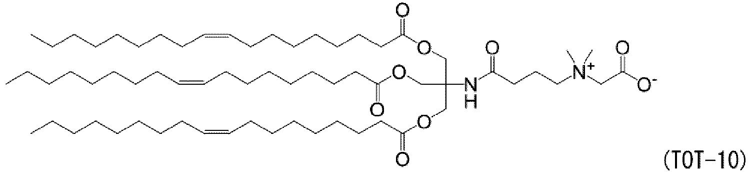

TOT-0 (366 mg, 0.40 mmol) was dissolved in anhydrous DCM (4 mL), and 4-(dimethylamino)butyric acid hydrochloride (83.8 mg, 0.50 mmol), DMAP (4.9 mg, 0.04 mmol), TEA (83.6 µL, 0.60 mmol), and EDCI (115 mg, 0.60 mmol) were added to the resulting mixture. The reaction mixture was stirred at 35°C overnight and then evaporated. The resulting residue was suspended with hexane/EtOAc (1 mL: 1.4 mL), washed with 0.5 N NaOH aq. (5 mL), and then dried over anhydrous Na₂SO₄. The resulting solution was filtered, and the filtrate was evaporated. The resulting residue was loaded on a normal-phase column (Sfär Silica HCD, manufactured by Biotage) and purified by flash chromatography with a gradient mobile phase of DCM and methanol. This gave 2-(4-(dimethylamino)butanamido)-2-((oleoyloxy)methyl)propane-1,3-diyl dioleate (compound 3) (341 mg, yield 83%) as a colorless oil.

Compound 3 (154 mg, 0.15 mmol) was dissolved in anhydrous DCM (1 mL), and bromoacetic acid (41.7 mg, 0.30 mmol) and N,N-diisopropylamine (DIPEA) (102 µL, 0.60 mmol) were added to the resulting mixture. The reaction mixture was stirred at 50°C for 2 hours under argon atmosphere and then evaporated. The resulting residue was loaded on a reverse-phase column (Sfär C18, Biotage) and purified by flash chromatography with a gradient mobile phase of water and 2-propanol. This gave TOT-10 (55.5 mg, yield 34%) as a colorless viscous oil.

### [Example 3]

3-(Dimethylamino)propionic acid was condensed to the amino group in TOT-0 synthesized in Example 1 to synthesize 2-((3-((1,3-bis(oleoyloxy)-2-((oleoyloxy)methyl)propan-2-yl)amino)-3-oxopropyl)dime thylammonio)acetate (TOT-11).

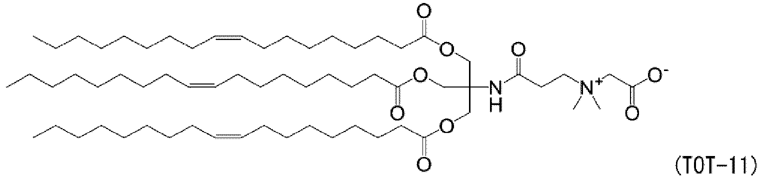

2-(3-(Dimethylamino)propanamido)-2-((oleoyloxy)methyl)propane- 1,3-diyl dioleate (compound 4) (245 mg, yield 60%) was obtained as a colorless oil in the same manner as in the synthesis of compound 3 in Example 2, except that 3-(dimethylamino)propionic acid hydrochloride (76.8 mg, 0.50 mmol) was used instead of 4-(dimethylamino)butyric acid hydrochloride.

Compound 4 (152 mg, 0.15 mmol) was dissolved in anhydrous DCM (1 mL), and bromoacetic acid (31.3 mg, 0.225 mmol) and DIPEA (76.5 µL, 0.45 mmol) were added to the resulting mixture. The reaction mixture was stirred at 40°C overnight under argon atmosphere, and then evaporated. The resulting residue was loaded on a reverse-phase column (Sfär C18, Biotage), and purified by flash chromatography with a gradient mobile phase of water and 2-propanol. This gave TOT-11 (85.8 mg, yield 53%) as a colorless viscous oil.

### [Example 4]

Fmoc-N-methyl-β-alanine was condensed to the amino group in TOT-0 synthesized in Example 1 to synthesize 2-((3-((1,3-bis(oleoyloxy)-2-((oleoyloxy)methyl)propan-2-yl)amino)-3-oxopropyl)(met hyl)ammonio)acetate (TOT-12).

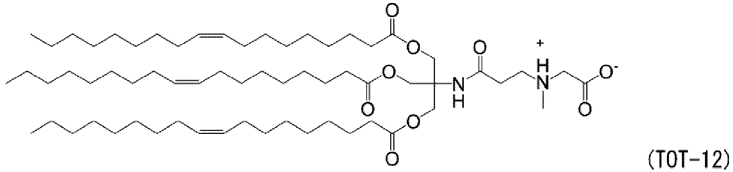

TOT-0 (914 mg, 1.00 mmol) was dissolved in ethanol (10 mL), and Fmoc-N-methyl-β-alanine (358 mg, 1.10 mmol) and DMT-MM (304 mg, 1.10 mmol) were added to the resulting mixture. The reaction mixture was stirred at 40°C overnight and then evaporated. The resulting residue was suspended with hexane/EtOAc (1 mL:11.20 mL), washed with 0.5 N NaOH aq. (5 mL), then washed with brine, and dried over anhydrous Na₂SO₄. The resulting solution was filtered, and the filtrate was evaporated.

The crude residue (colorless oil) was dissolved in anhydrous DCM (4 mL), and piperidine (1 mL) was added to the resulting mixture. The reaction mixture was stirred at ambient temperature for 10 minutes and then evaporated. The resulting residue was loaded on a normal-phase column (Sfär Silica HCD, manufactured by Biotage) and purified by flash chromatography with a gradient mobile phase of DCM and methanol. This gave a colorless viscous oil (388 mg, yield 39%).

The resulting colorless oil (388 mg, 0.388 mmol) was dissolved in anhydrous DCM (5 mL), and bromoacetic acid (80.9 mg, 0.582 mmol) and DIPEA (198 µL, 1.16 mmol) were added to the resulting mixture. The reaction mixture was stirred at 35°C overnight under argon atmosphere, and then evaporated. The resulting residue was loaded on a reverse-phase column (Sfär C18, Biotage), and purified by flash chromatography with a gradient mobile phase of water and 2-propanol. This gave TOT-12 (152.4 mg, yield 37%) as a colorless viscous oil.

### [Example 5]

4-tert-Butyl-N-(tert-butoxycarbonyl)-L-aspartic acid was condensed to the amino group in TOT-0 synthesized in Example 1 to synthesize (S)-3-amino-4-((1,3-bis(oleoyloxy)-2-((oleoyloxy)methyl)propan-2-yl)amino)-4-oxobut anoic acid (TOT-13).

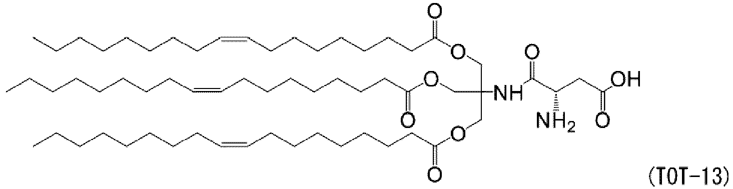

TOT-0 (366 mg, 0.40 mmol) was dissolved in ethanol (10 mL), and 4-tert-butyl-N-(tert-butoxycarbonyl)-L-aspartic acid (173 mg, 0.44 mmol) and DMT-MM (122 mg, 0.44 mmol) were added to the resulting mixture. The reaction mixture was stirred at ambient temperature overnight and then evaporated. The resulting residue was suspended with EtOAc (4 mL), washed with 0.5 N NaOH aq. (5 mL), and then dried over anhydrous Na₂SO₄. The resulting solution was filtered, and the filtrate was evaporated. The residue was dissolved in anhydrous DCM (2.8 mL), TFA (0.8 mL) and TIPS (0.4 mL) were added thereto, and the mixture was stirred at ambient temperature overnight. The resulting reaction mixture was diluted with EtOAc, and the organic phase was washed with 1 N aqueous NaOH solution, water, and brine, and dried over anhydrous Na₂SO₄. The resulting solution was filtered, and the filtrate was evaporated. The resulting residue was loaded on a normal-phase column (Sfär Silica HCD, manufactured by Biotage) and purified by flash chromatography with a gradient mobile phase of hexane and EtOAc. This gave TOT-13 (180 mg, yield 44%) as a colorless viscous oil.

### [Example 6]

1-tert-Butyl-N-(tert-butoxycarbonyl)-L-aspartic acid was condensed to the amino group in TOT-0 synthesized in Example 1 to synthesize N4-(1,3-bis(oleoyloxy)-2-((oleoyloxy)methyl)propan-2-yl)-L-asparagine (TOT-14).

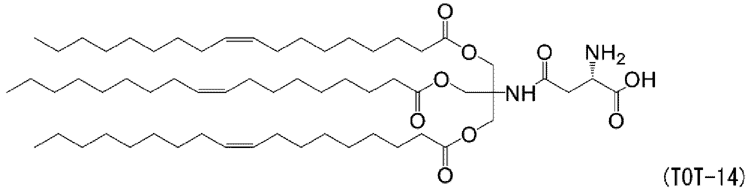

TOT-0 (366 mg, 0.40 mmol) was dissolved in ethanol (4 mL), and 1-tert-butyl-N-(tert-butoxycarbonyl)-L-aspartic acid (127 mg, 0.44 mmol) and DMT-MM (122 mg, 0.44 mmol) were added to the resulting mixture. The reaction mixture was stirred at ambient temperature overnight and then evaporated. The resulting residue was suspended with EtOAc (4 mL), washed with 0.5 N NaOH aq. (5 mL), and then dried over anhydrous Na₂SO₄. The residue was dissolved in anhydrous DCM (2.8 mL), and TFA (0.8 mL) and TIPS (0.4 mL) were added thereto. The resulting reaction mixture was diluted with EtOAc, and the organic phase was washed with 1 N aqueous NaOH solution, water, and brine, and dried over anhydrous Na₂SO₄. The resulting solution was filtered, and the filtrate was evaporated. The residue was loaded on a normal-phase column (Sfär Silica HCD, manufactured by Biotage) and purified by flash chromatography with a gradient mobile phase of hexane and EtOAc. This gave TOT-14 (199 mg, yield 48%) as a colorless viscous oil.

### [Example 7]

1-tert-Butyl-N-(tert-butoxycarbonyl)-L-glutamic acid was condensed to the amino group in TOT-0 synthesized in Example 1 to synthesize N5-(1,3-bis(oleoyloxy)-2-((oleoyloxy)methyl)propan-2-yl)-L-glutamine (TOT-15).

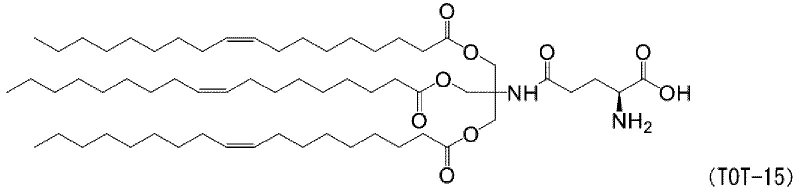

TOT-0 (366 mg, 0.40 mmol) was dissolved in ethanol (4 mL), and 1-tert-butyl-N-(tert-butoxycarbonyl)-L-glutamic acid (133.4 mg, 0.44 mmol) and DMT-MM (122 mg, 0.44 mmol) were added to the resulting mixture. The resulting reaction mixture was stirred at ambient temperature overnight and then evaporated. The resulting residue was suspended with EtOAc (4 mL), washed with 0.5 N NaOH aq. (5 mL), and then dried over anhydrous Na₂SO₄. The resulting solution was filtered, the filtrate was evaporated, then dissolved in anhydrous DCM (2.8 mL), and TFA (0.8 mL) and TIPS (0.4 mL) were added thereto. The resulting reaction mixture was stirred at ambient temperature overnight, then diluted with EtOAc, and the organic phase was washed with 1 N aqueous NaOH solution, water, and brine, and then dried over anhydrous Na₂SO₄. The resulting residue was loaded on a normal-phase column (Sfär Silica HCD, manufactured by Biotage) and purified by flash chromatography with a gradient mobile phase of hexane and EtOAc. This gave TOT-15 (141 mg, yield 34%) as a colorless viscous oil.

### [Example 8]

The effect of TOT-10 synthesized in Example 2 on the stability of the DOPC membrane was examined. Specifically, a lipid membrane having a lipid composition of DOPC/TOT-10=50/50 (mol%) and a lipid membrane having a lipid composition of DOPC/DOPE=50/50 (mol%) were prepared, and proton-decoupled ³¹P NMR spectra were obtained by ³¹P NMR spectroscopy.

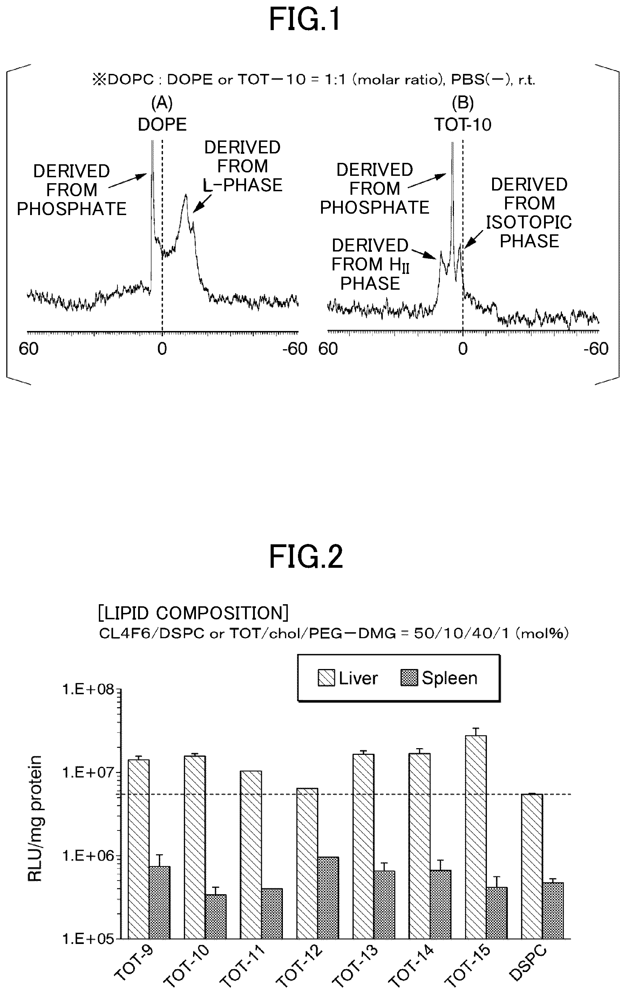

Fig. 1(A) shows the ³¹P NMR spectrum of the DOPC/DOPE membrane, and Fig. 1(B) shows the ³¹P NMR spectrum of the DOPC/TOT-10 membrane. In the DOPC/DOPE membrane, a peak derived from the lamellar phase was observed (Fig. 1(A)), whereas in the DOPC/TOT-10 membrane, a peak derived from the hexagonal phase or isotropic phase was observed instead of a peak derived from the lamellar phase (Fig. 1(B)). These results indicated that TOT-10 reduces the membrane stability of a lipid membrane containing phosphatidylcholine and can disrupt the membrane.

### [Example 9]

Lipid nanoparticles were prepared using TOT-9 to TOT-15 synthesized in Examples 1 to 7 as constituent lipids.

A pH-sensitive cationic lipid, cholesterol, and PEG-DMG were used as lipids other than TOT-9 to TOT-15. CL4F6 was used as the pH-sensitive cationic lipid. DSPC was also used instead of TOT.

Specifically, CL4F6, TOT-9 to TOT-15, cholesterol, and PEG-DMG were used in a composition of CL4F6/TOT/chol/PEG-DMG=50/10/40/1 (mol%) to prepare lipid nanoparticles loaded with Fluc mRNA (Fluc mRNA-loaded lipid nanoparticles) by the alcohol dilution method. Further, Fluc mRNA-loaded lipid nanoparticles were prepared in the same manner except that DSPC was used instead of TOT with a composition of CL4F6/DSPC/chol/PEG-DMG=50/10/40/1 (mol%).

Next, each of the prepared Fluc mRNA-loaded lipid nanoparticles was administered to BALB/c mice, and Fluc activity (RLU/mg protein) was measured. The results are shown in Fig. 2. Fluc activity was confirmed in the liver and spleen of mice administered with any of the lipid nanoparticles. From this result, it was confirmed that the lipid nanoparticles using TOT-9 to TOT-15 as constituent lipids are useful as gene carriers targeting the liver and spleen. Further, all the TOT-containing Fluc mRNA-loaded lipid nanoparticles showed higher Fluc activity in the liver than that of the DSPC-containing Fluc mRNA-loaded lipid nanoparticles.

### [Example 10]

The gene expression efficiency in the liver when lipid nanoparticles containing TOT-15 synthesized in Example 7 and a pH-sensitive cationic lipid as constituent lipids were administered by intravenous injection was examined.

Specifically, a pH-sensitive cationic lipid, TOT-15, cholesterol, and PEG-DMG were used in a composition of pH-sensitive cationic lipid/TOT-15/chol/PEG-DMG=50/10/40/1.5 (mol%) to prepare Fluc mRNA-loaded lipid nanoparticles by the alcohol dilution method. As the pH-sensitive cationic lipid, CL4F6, CL4H6, DLin-MC3-DMA, or ALC-0315 was used. Further, Fluc mRNA-loaded lipid nanoparticles having a composition of pH-sensitive cationic lipid/DSPC/chol/PEG-DMG=50/10/40/1.5 (mol%) were prepared in the same manner except that DSPC was used instead of TOT.

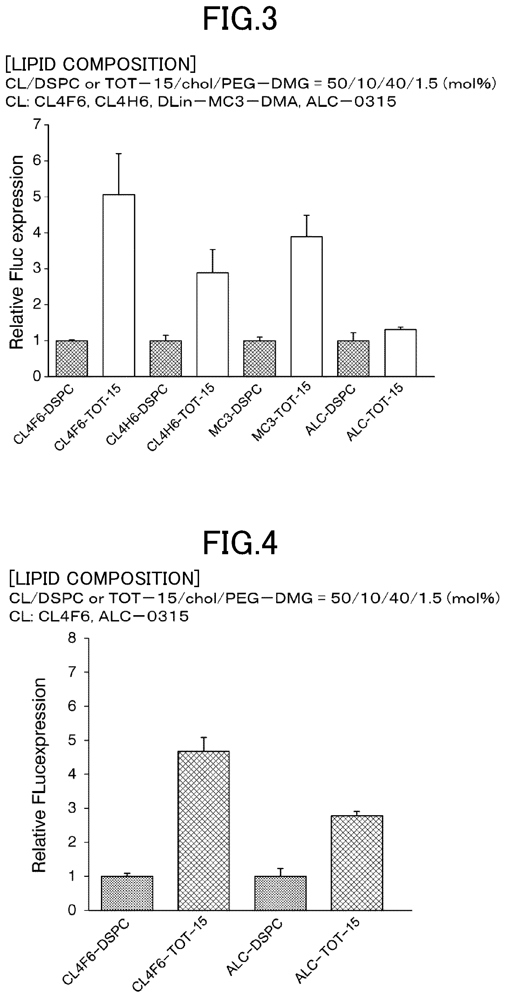

Next, each of the prepared Fluc mRNA-loaded lipid nanoparticles was administered to BALB/c mice by intravenous injection, and Fluc activity (RLU/mg protein) was measured. The results are shown in Fig. 3. In the Fluc mRNA-loaded lipid nanoparticles containing CL4F6, CL4H6, or DLin-MC3-DMA, the Fluc activity in the liver was clearly higher in the lipid nanoparticles containing TOT-15 than in the lipid nanoparticles containing DSPC. Further, in the Fluc mRNA-loaded lipid nanoparticles containing ALC-0315, a tendency was observed that the Fluc activity in the liver was better in the lipid nanoparticles containing TOT-15 than in the lipid nanoparticles containing DSPC.

### [Example 11]

The gene expression efficiency in the quadriceps femoris muscle when lipid nanoparticles containing TOT-15 synthesized in Example 7 and a pH-sensitive cationic lipid as constituent lipids were administered by intramuscular injection was examined.

Specifically, among the Fluc mRNA-loaded lipid nanoparticles prepared in Example 10, Fluc mRNA-loaded lipid nanoparticles containing CL4F6 or ALC-0315 were administered to BALB/c mice by intramuscular injection, and Fluc activity (RLU/mg protein) was measured. The results are shown in Fig. 4. In the Fluc mRNA-loaded lipid nanoparticles containing CL4F6 or ALC-0315, the Fluc activity in the quadriceps femoris muscle was clearly higher in the lipid nanoparticles containing TOT-15 than in the lipid nanoparticles containing DSPC even when administered by intramuscular injection.

### [Example 12]

The gene expression efficiency in the brain when lipid nanoparticles containing TOT-15 synthesized in Example 7 and a pH-sensitive cationic lipid as constituent lipids were administered by intracerebral injection was examined.

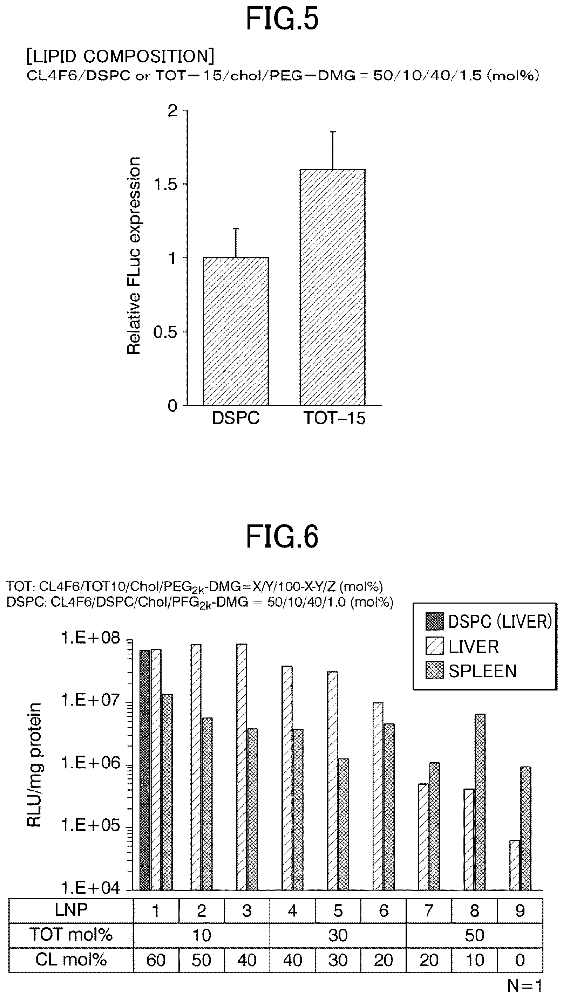

Specifically, among the Fluc mRNA-loaded lipid nanoparticles prepared in Example 10, Fluc mRNA-loaded lipid nanoparticles containing CL4F6 were administered to BALB/c mice by intracerebral injection, and Fluc activity (RLU/mg protein) was measured. The results are shown in Fig. 5. In the Flue mRNA-loaded lipid nanoparticles containing CL4F6, the Flue activity in the brain was clearly higher in the lipid nanoparticles containing TOT-15 than in the lipid nanoparticles containing DSPC even when administered by intracerebral injection.

### [Example 13]

The effect of the proportion of the ionic neutral lipid in the constituent lipids of lipid nanoparticles on the delivery efficiency of the lipid nanoparticles to target cells in vivo was examined. TOT-10 synthesized in Example 2 was used as the ionic neutral lipid. A pH-sensitive cationic lipid, cholesterol, and PEG-DMG were used as lipids other than the ionic neutral lipid. CL4F6 was used as the pH-sensitive cationic lipid. DSPC was also used instead of TOT-10.

Specifically, CL4F6, TOT-10, cholesterol, and PEG-DMG were used in a composition of CL4F6/TOT-10/chol/PEG-DMG=x/y/100-x-y/z (mol%) to prepare lipid nanoparticles loaded with Flue mRNA (Flue mRNA-loaded lipid nanoparticles) (LNP-1 to 9) by the alcohol dilution method. The average particle size (number average value), ζ-average particle size, PdI, and encapsulation rate of the prepared lipid nanoparticles were measured. The results are shown in Table 1. Further, Fluc mRNA-loaded lipid nanoparticles (LNP-10) were prepared in the same manner except that DSPC was used instead of TOT with a composition of CL4F6/DSPC/chol/PEG-DMG=50/10/40/1 (mol%).

**Table 1**

| LNP | CL4F6 (Y) [mol%] | TOT-10 (X) [mol%] | Chol [mol%] | PEG (Z) [mol%] | ζ-Average Particle Size [nm] | Number Average Particle Size [nm] | Encapsulation Rate [%] | PdI |
|---|---|---|---|---|---|---|---|---|
| 1 | 60 | 10 | 30 | 0.6 | 156 | 97 | 74.4 | 0.17 |
| 2 | 50 | 10 | 40 | 0.6 | 133 | 60 | 93.4 | 0.24 |
| 3 | 40 | 10 | 50 | 1 | 133 | 49 | 92.1 | 0.23 |
| 4 | 40 | 30 | 30 | 0.6 | 124 | 82 | 91.7 | 0.19 |
| 5 | 30 | 30 | 40 | 1 | 110 | 67 | 96.6 | 0.24 |
| 6 | 20 | 30 | 50 | 1 | 160 | 97 | 90.1 | 0.16 |
| 7 | 20 | 50 | 30 | 1 | 117 | 39 | 95.3 | 0.21 |
| 8 | 10 | 50 | 40 | 0.6 | 170 | 121 | 93.5 | 0.15 |
| 9 | 0 | 50 | 50 | 0.6 | 171 | 108 | 61.9 | 0.15 |

Next, each of the prepared Fluc mRNA-loaded lipid nanoparticles was administered to BALB/c mice (4 weeks old, female) (0.1 mg RNA/kg), the liver and spleen of the mice were collected 6 hours after administration, and Fluc activity (expression level of Fluc protein) (RLU/mg protein) was measured (N=1). The results are shown in Fig. 6. The activity in the liver increased in proportion to the CL4F6 content, and when the CL4F6 content was the same, the Fluc activity decreased as the TOT-10 content increased. In particular, the expression efficiency of Fluc in the liver was preferable in Flue mRNA-loaded lipid nanoparticles having a TOT-10 content of 30 mol% or less, and most preferable in Fluc mRNA-loaded lipid nanoparticles having a TOT-10 content of about 10 mol%.

### [Example 14]

The membrane disruption ability of lipid nanoparticles prepared using TOT-9 to TOT-15 synthesized in Examples 1 to 7 as constituent lipids was examined by a hemolysis test.

A pH-sensitive cationic lipid, cholesterol, and PEG-DMG were used as lipids other than TOT-9 to TOT-15. CL4F6 was used as the pH-sensitive cationic lipid. DSPC was also used instead of TOT.

### <Lipid Nanoparticles>

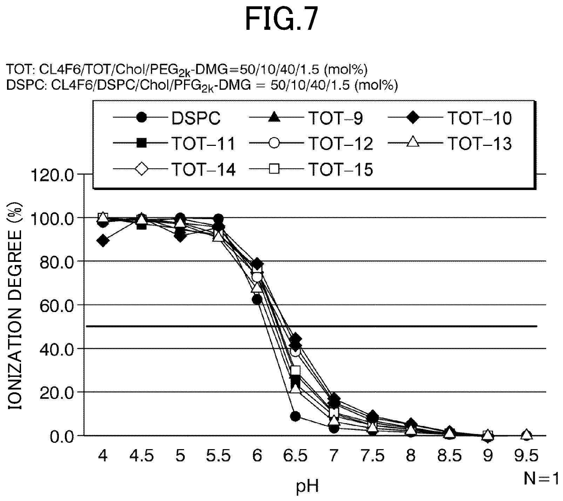

Specifically, CL4F6, TOT-9 to TOT-15, cholesterol, and PEG-DMG were used in a composition of CL4F6/TOT/chol/PEG-DMG=50/10/40/1.5 (mol%) to prepare lipid nanoparticles by the alcohol dilution method. Further, lipid nanoparticles were prepared in the same manner except that DSPC was used instead of TOT with a composition of CL4F6/DSPC/chol/PEG-DMG=50/10/40/1.5 (mol%). The average particle size (number average value), ζ-average particle size, PdI, ionization degree at each pH, and apparent pKa of the prepared lipid nanoparticles were measured. The measurement results are shown in Table 2 and Fig. 7.

**Table 2**

| Neutral Lipid | ζ-Average Particle Size [nm] | Number Average Particle Size [nm] | PdI | pKa |
|---|---|---|---|---|
| DSPC | 128 | 67 | 0.21 | 6.12 |
| TOT-9 | 157 | 109 | 0.15 | 6.27 |
| TOT-10 | 141 | 73 | 0.28 | 6.39 |
| TOT-11 | 131 | 83 | 0.17 | 6.41 |
| TOT-12 | 135 | 86 | 0.18 | 6.34 |
| TOT-13 | 125 | 81 | 0.12 | 6.19 |
| TOT-14 | 141 | 76 | 0.23 | 6.24 |
| TOT-15 | 119 | 61 | 0.24 | 6.29 |

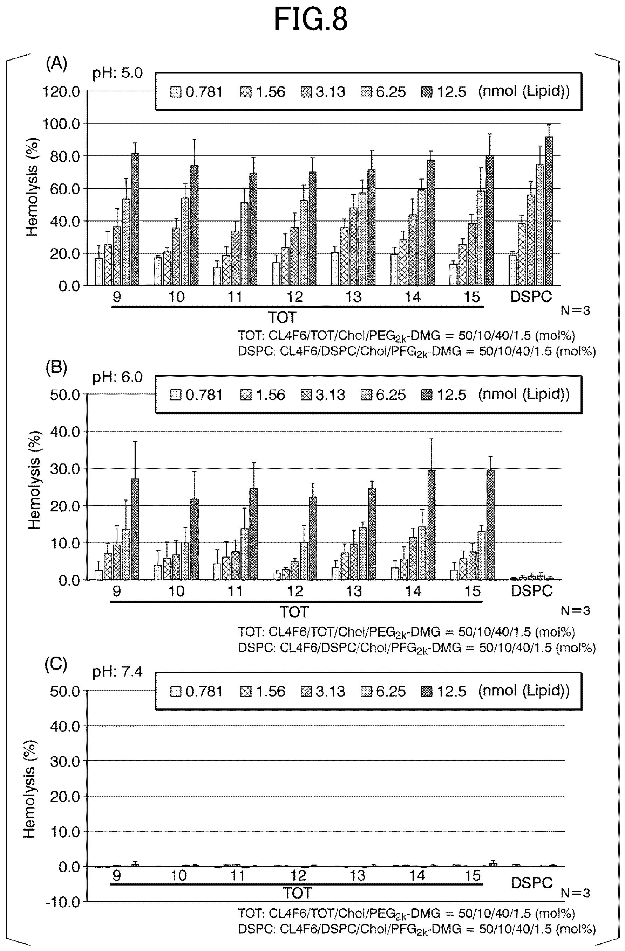

A hemolysis test was performed on each of the lipid nanoparticles, and the hemolytic activity at pH 5.0, 6.0, and 7.4 was measured. At pH 5.0, all the lipid nanoparticles underwent membrane disruption and showed the same level of hemolytic activity (Fig. 8(A)). Further, at pH 7.4, no membrane disruption occurred in any of the lipid nanoparticles (Fig. 8(C)). At pH 6.0, no membrane disruption occurred in the lipid nanoparticles containing DSPC, whereas all the lipid nanoparticles containing TOT-9 to TOT-15 showed hemolytic activity of about 30% at maximum (Fig. 8(B)). These results indicated that TOT-9 to TOT-15 promote membrane fusion in early endosomes, suggesting that this membrane fusion promoting ability achieves high delivery efficiency to target cells.

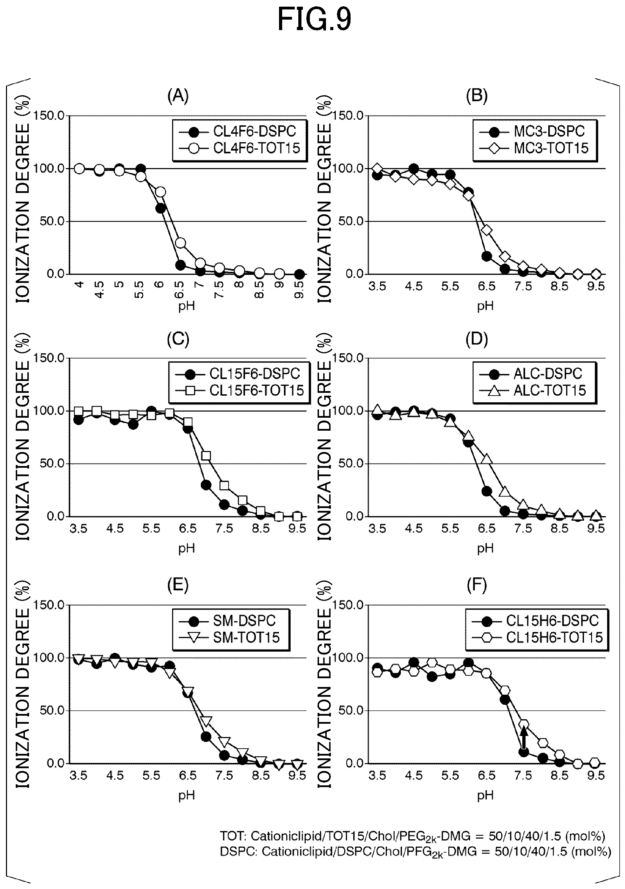

Lipid nanoparticles were prepared in the same manner as above using CL4F6, ALC-0315, MC3, SM-102, CL15F6, or CL15H6 as a cationic lipid and TOT-15 or DSPC as an ionic neutral lipid, and the ionization degree at each pH and apparent pKa were measured. The measurement results are shown in Table 3 and Fig. 9. Fig. 9(A) shows the results of lipid nanoparticles using CL4F6, Fig. 9(B) shows the results using MC3, Fig. 9(C) shows the results using CL15F6, Fig. 9(D) shows the results using ALC-0315, Fig. 9(E) shows the results using SM-102, and Fig. 9(F) shows the results using CL15H6. In the lipid nanoparticles containing any cationic lipid, the pKa was similar between those containing TOT-15 and those containing DSPC (Table 3), but the ionization degree was higher in the case of containing TOT-15 (for example, "CL4F6-TOT15" in Fig. 9(A)) than in the case of containing DSPC (for example, "CL4F6-DSPC" in Fig. 9(A)) in the high pH region in the latter half of the sigmoid curve (Fig. 9).

**Table 3**

| Cationic Lipid | Ionic Neutral Lipid | pKa |
|---|---|---|
| CL4F6 | DSPC | 6.23 |
| | TOT-15 | 6.38 |
| ALC-0315 | DSPC | 6.22 |
| | TOT-15 | 6.57 |
| MC3 | DSPC | 6.23 |
| | TOT-15 | 6.38 |
| SM-102 | DSPC | 6.71 |
| | TOT-15 | 6.83 |
| CL15F6 | DSPC | 6.81 |
| | TOT-15 | 7.13 |
| CL15H6 | DSPC | 7.13 |
| | TOT-15 | 7.34 |

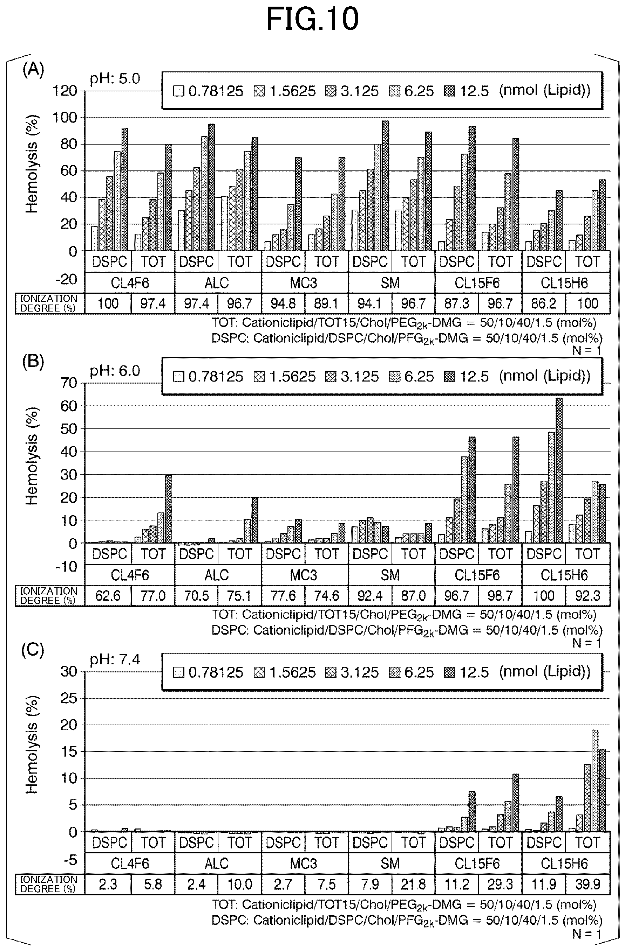

A hemolysis test was performed on each of the lipid nanoparticles, and the hemolytic activity at pH 5.0, 6.0, and 7.4 was measured. At pH 5.0, lipid nanoparticles containing TOT-15 and lipid nanoparticles containing DSPC showed the same level of hemolytic activity regardless of the type of cationic lipid (Fig. 10(A)). On the other hand, at pH 6.0, when CL4F6 or ALC was used as the cationic lipid, no membrane disruption occurred in the lipid nanoparticles containing DSPC, whereas hemolytic activity was observed in the lipid nanoparticles containing TOT-15 (Fig. 10(B)). When MC3, SM, CL15F6, or CL15H6 was used as the cationic lipid, the lipid nanoparticles containing TOT-15 showed hemolytic activity equal to or lower than that of the lipid nanoparticles containing DSPC (Fig. 10(B)). At pH 7.4, membrane disruption occurred in the lipid nanoparticles using CL15F6 or CL15H6, and the hemolytic activity was higher in the case of containing TOT-15 than in the case of containing DSPC (Fig. 10(C)).

### [Example 15]

The delivery efficiency of lipid nanoparticles using TOT-15 synthesized in Example 7 to the liver was compared with that of lipid nanoparticles using DSPC. A pH-sensitive cationic lipid, cholesterol, and PEG-DMG were used as lipids other than TOT-15 and DSPC. CL4F6 was used as the pH-sensitive cationic lipid.

Specifically, CL4F6, TOT-15, cholesterol, and PEG-DMG were used in a composition of CL4F6/TOT-15/chol/PEG-DMG=50/10/40/1.5 (mol%) to prepare lipid nanoparticles loaded with Flue mRNA (Fluc mRNA-loaded lipid nanoparticles) by the alcohol dilution method. Further, Fluc mRNA-loaded lipid nanoparticles having a composition of CL4F6/DSPC/chol/PEG-DMG=50/10/40/1.5 (mol%) were prepared in the same manner except that DSPC was used instead of TOT.

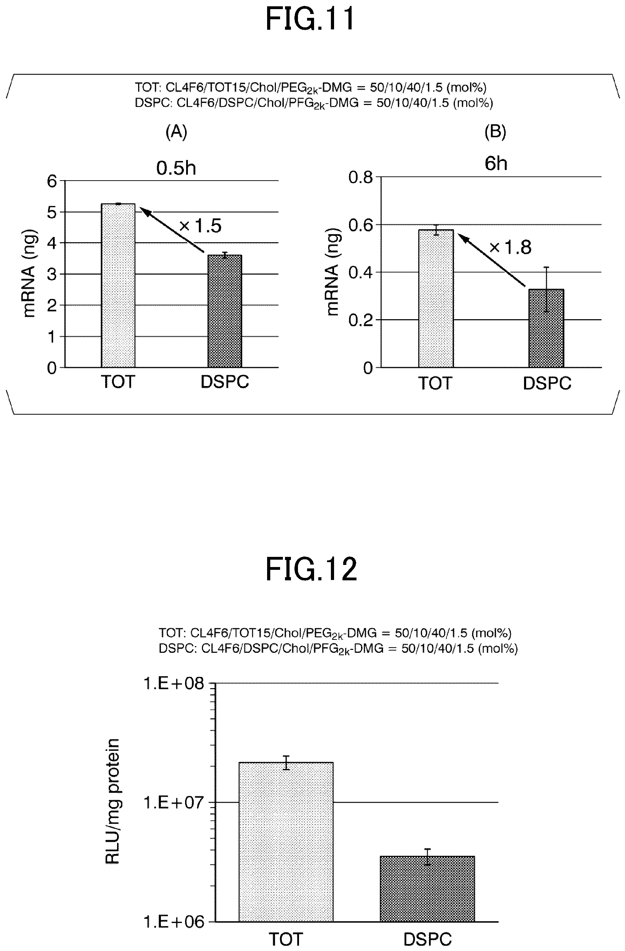

Next, each of the prepared Fluc mRNA-loaded lipid nanoparticles was administered to BALB/c mice (4 weeks old, female) (0.01 mg RNA/kg), the liver of the mice was collected 0.5 hours or 6 hours after administration, and the amount of Fluc mRNA taken up into the liver was measured by qRT-PCR. The measurement results are shown in Fig. 11. In both 0.5 hours (Fig. 11(A)) and 6 hours (Fig. 11(B)) after administration, the lipid nanoparticles containing TOT-15 took up about twice as much mRNA into the liver as the lipid nanoparticles containing DSPC. From these results, it was confirmed that the lipid nanoparticles containing TOT-15 deliver more mRNA than the lipid nanoparticles containing DSPC.

Further, Fluc activity (expression level of Fluc protein) (RLU/mg protein) 6 hours after administration of the Fluc mRNA-loaded lipid nanoparticles was measured. The results are shown in Fig. 12. In the liver, the lipid nanoparticles containing TOT-15 showed about 6.1 times higher Fluc activity of mRNA than the lipid nanoparticles containing DSPC. The difference in the amount of mRNA transferred to the liver between the lipid nanoparticles containing TOT-15 and the lipid nanoparticles containing DSPC is smaller than the difference in Fluc activity between the two, suggesting that the increased activity by the lipid nanoparticles containing TOT-15 is not only due to the increased amount of transfer to target cells but also due to the high escape efficiency from endosomes.

### [Example 16]

Lipid nanoparticles using TOT-15 synthesized in Example 7 were used as a DDS carrier for genome editing. Lipid nanoparticles using DSPC were used as a control. A pH-sensitive cationic lipid, cholesterol, and PEG-DMG were used as lipids other than TOT-15 and DSPC. CL4F6 was used as the pH-sensitive cationic lipid.

Specifically, first, an RNA solution was prepared so that the mass ratio of Cas9-mRNA (TriLink, 5moU modified) to gRNA targeting transthyretin (TTR) was 1:2. Using this RNA solution together with CL4F6, TOT-15, cholesterol, and PEG-DMG in a composition of CL4F6/TOT-15/chol/PEG-DMG=50/10/40/1.5 (mol%), lipid nanoparticles loaded with Cas9-mRNA and TTR-gRNA (TTR-targeted genome editing lipid nanoparticles) were prepared by the alcohol dilution method. Lipid nanoparticles (TTR-targeted genome editing lipid nanoparticles) were prepared in the same manner using DSPC instead of TOT-15, and used as a control. The average particle size (number average value), ζ-average particle size, PdI, and encapsulation rate of the prepared lipid nanoparticles were measured. The results are shown in Table 4.

**Table 4**

| Ionic Neutral Lipid | ζ-Average Particle Size [nm] | Number Average Particle Size [nm] | Encapsulation Rate [%] | PdI |
|---|---|---|---|---|
| TOT-15 | 193 | 136 | 100.7 | 0.12 |
| DSPC | 168 | 61 | 91.1 | 0.18 |

Next, each of the prepared TTR-targeted genome editing lipid nanoparticles was administered to BALB/c mice (4 weeks old, female) via the tail vein (1.0 mg mRNA/kg), the mice were euthanized 1 week after administration, and blood was collected from the inferior vena cava. The blood was allowed to stand at room temperature for 1 hour, centrifuged at 25°C and 1,000×g for 15 minutes, and the supernatant was collected to obtain serum. The TTR concentration in the serum was measured using a commercially available ELISA kit (Prealbumin ELISA Kit Mouse, manufactured by Aviva Systems Biology). Calibration curve samples were prepared by diluting 500 ng/mL solution stepwise to 250, 125, 62.5, 31.25, and 15.63 ng/mL, with 300 µL of each solution. Serum samples were prepared by diluting the serum 10,000 times. 100 µL each of the calibration curve sample and the serum sample was applied to a plate and incubated at 25°C for 30 minutes. The wells were washed 4 times, 100 µL of Enzyme-Antibody Conjugate was applied to each well, the plate was shielded from light with aluminum foil, and incubated at 25°C for 20 minutes. The wells were washed 4 times again, 100 µL of TMB Substrate Solution (manufactured by Aviva Systems Biology) was applied to each well, the plate was shielded from light with aluminum foil, and incubated at 25°C for 10 minutes, then 100 µL of Stop Solution (manufactured by Aviva Systems Biology) was applied, and the absorbance at 450 nm was measured using a fluorescence spectrophotometer (Varioskan Lux, manufactured by Thermo Fisher Scientific).

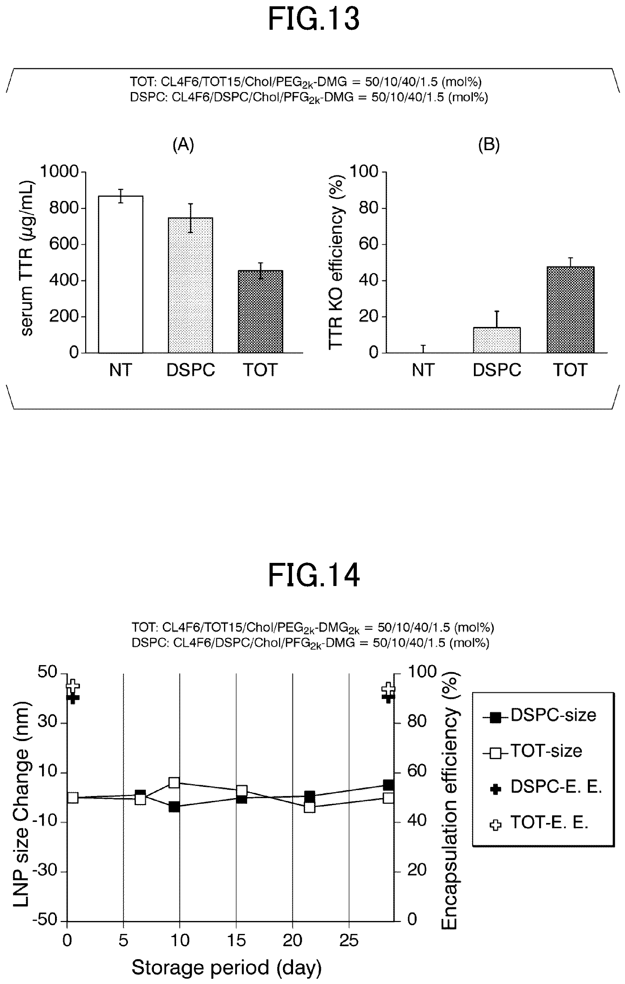

Fig. 13 shows the measurement results of the TTR amount in serum (Fig. 13(A)) and the TTR knockdown efficiency (Fig. 13(B)). The TTR knockdown efficiency was about 3.4 times higher in the lipid nanoparticles containing TOT-15 than in the lipid nanoparticles containing DSPC.

### [Example 17]

The storage stability of lipid nanoparticles using TOT-15 synthesized in Example 7 was examined. Lipid nanoparticles using DSPC were used as a control. A pH-sensitive cationic lipid, cholesterol, and PEG-DMG were used as lipids other than TOT-15 and DSPC. CL4F6 was used as the pH-sensitive cationic lipid.

Specifically, CL4F6, TOT-15, cholesterol, and PEG-DMG were used in a composition of CL4F6/TOT/chol/PEG-DMG=50/10/40/1.5 (mol%) to prepare lipid nanoparticles loaded with Flue mRNA (Fluc mRNA-loaded lipid nanoparticles) by the alcohol dilution method. Further, lipid nanoparticles loaded with Flue mRNA (Fluc mRNA-loaded lipid nanoparticles) were prepared in the same manner except that DSPC was used instead of TOT-15 with a composition of CL4F6/DSPC/chol/PEG-DMG=50/10/40/1.5 (mol%), and stored at 4°C for 4 weeks. The average particle size (number average value), PdI, and encapsulation rate of the prepared lipid nanoparticles were measured over time. The measurement results at the time of preparation are shown in Table 5. The results of time-course measurement of average particle size (number average value) and encapsulation rate are shown in Fig. 14. As shown in Fig. 14, storage at 4°C for 4 weeks did not affect the particle size or encapsulation rate of the lipid nanoparticles containing TOT-15.

**Table 5**

| Ionic Neutral Lipid | Number Average Particle Size [nm] | PdI | Encapsulation Rate[%] |
|---|---|---|---|
| TOT-15 | 58 | 0.23 | 95.1 |
| DSPC | 80 | 0.21 | 90.5 |

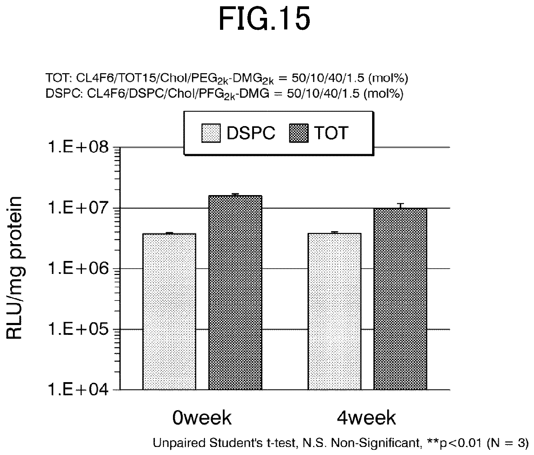

Further, each of the Fluc mRNA-loaded lipid nanoparticles immediately after production and after storage for 4 weeks was administered to BALB/c mice (4 weeks old, female) (0.01 mg RNA/kg), the liver of the mice was collected 6 hours after administration, and Fluc activity (expression level of Fluc protein) (RLU/mg protein) was measured. The results are shown in Fig. 15. As shown in Fig. 15, storage at 4°C for 4 weeks did not affect the Fluc activity of the lipid nanoparticles containing TOT-15, indicating that the lipid nanoparticles can be stably stored for at least one month.

### [Example 18]

Lipid nanoparticles using TOT-15 synthesized in Example 7 were used as a DDS carrier for GFP expression, and GFP expression in living tissue was evaluated. Lipid nanoparticles using DSPC were used as a control. A pH-sensitive cationic lipid, cholesterol, and PEG-DMG were used as lipids other than TOT-15 and DSPC. CL4F6 was used as the pH-sensitive cationic lipid.

CL4F6, TOT-15, cholesterol, and PEG-DMG were used in a composition of CL4F6/TOT/chol/PEG-DMG=50/10/40/1.5 (mol%), 50/20/30/1.5 (mol%), or 50/30/20/1.5 (mol%) to prepare lipid nanoparticles loaded with EGFP-mRNA (TriLink, 5moU modified) (EGFP mRNA-loaded lipid nanoparticles) by the alcohol dilution method. Lipid nanoparticles loaded with EGFP-mRNA (EGFP-mRNA-loaded lipid nanoparticles) were prepared in the same manner except that DSPC was used instead of TOT-15 with using a composition of CL4F6/DSPC/chol/PEG-DMG=50/10/40/1.5 (mol%).

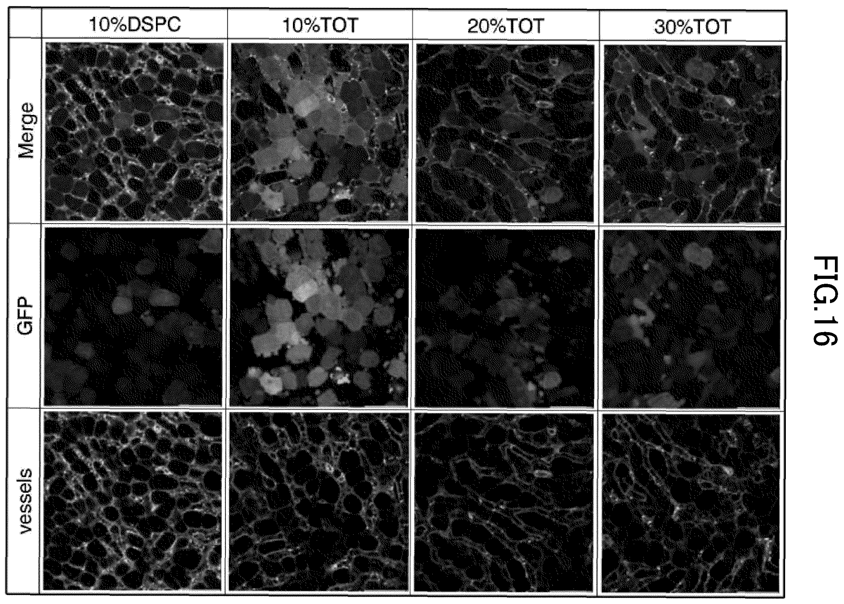

Each EGFP mRNA-loaded lipid nanoparticle was administered to BALB/c mice (4 weeks old, female) via the tail vein (1.00 mg RNA/kg). Ten minutes before tissue collection, DyLight-649 Lycopersicon (Tomato) Lectin was administered intravenously at 40 µg/mouse to fluorescently label vascular endothelial cells. The mice were euthanized 24 hours after administration of the EGFP mRNA-loaded lipid nanoparticles, and tissues were collected. To stain nuclei, a part of the tissue was immersed in PBS(-) containing 1 µg/mL Hoechst 33342, cooled on ice, and incubated for 30 minutes under light shielding. After washing the stained tissue with PBS(-), the tissue was observed with a confocal laser scanning microscope. Microscopic images are shown in Fig. 16. As a result, a tendency was observed that the EGFP expression activity was improved in cells other than hepatocytes as the proportion of TOT-15 in the constituent lipids of the lipid nanoparticles increased.

## Claims

1. An ionic neutral lipid comprising a compound represented by the following formula (I): 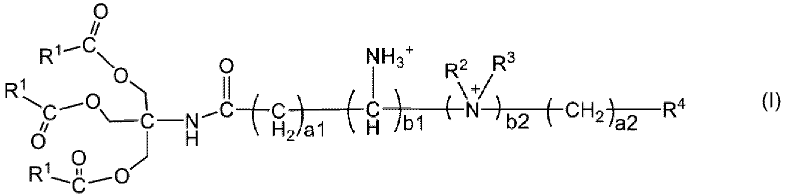 wherein, in formula (I), R¹ is a hydrocarbon group having 1 to 22 carbon atoms; three R¹ groups in one molecule may be the same as or different from each other; a1 and a2 are each independently an integer of 0 to 4; b1 and b2 are 0 or 1 satisfying b1+b2=1; R² and R³ are each independently a hydrogen atom or an alkyl group having 1 to 3 carbon atoms; and R⁴ is an anionic group.

2. The ionic neutral lipid according to claim 1, wherein R⁴ in the general formula (I) is a carboxylic acid group, a sulfonic acid group, a sulfate ester group, or a phosphate group.

3. The ionic neutral lipid according to claim 1, wherein the three R¹ groups in one molecule are all the same.

4. The ionic neutral lipid according to claim 1, wherein R¹ is a hydrocarbon group having 15 to 22 carbon atoms.

5. The ionic neutral lipid according to claim 1, which is a compound represented by any one of the following general formulas (Z-9) to (Z-15): 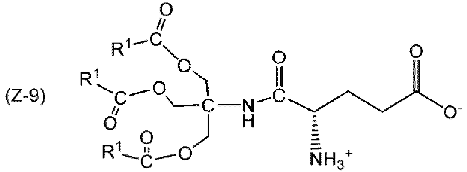 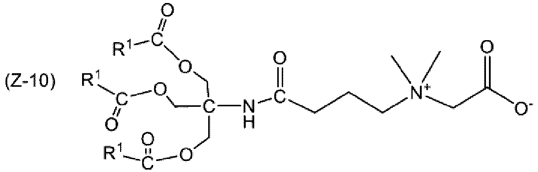 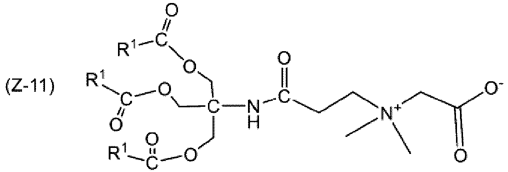 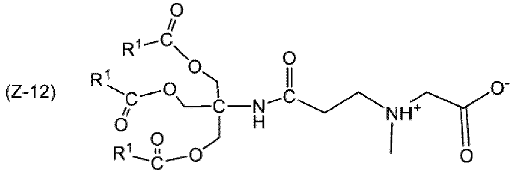 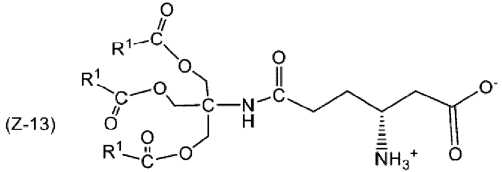 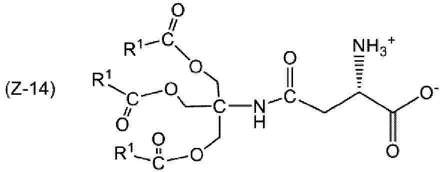 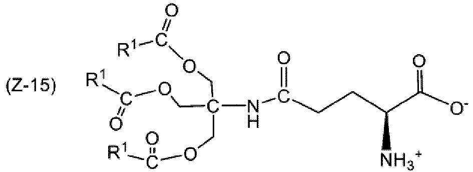 wherein R¹ is the same as in the general formula (I).

6. The ionic neutral lipid according to claim 1, which is (S)-4-amino-5-((1,3-bis(oleoyloxy)-2-((oleoyloxy)methyl)propan-2-yl)amino)-5-oxopen tanoic acid, 2-((4-((1,3-bis(oleoyloxy)-2-((oleoyloxy)methyl)propan-2-yl)amino)-4-oxobutyl)dimeth ylammonio)acetate, 2-((3-((1,3-bis(oleoyloxy)-2-((oleoyloxy)methyl)propan-2-yl)amino)-3-oxopropyl)dime thylammonio)acetate, 2-((3-((1,3-bis(oleoyloxy)-2-((oleoyloxy)methyl)propan-2-yl)amino)-3-oxopropyl)(met hyl)ammonio)acetate, (S)-3-amino-4-((1,3-bis(oleoyloxy)-2-((oleoyloxy)methyl)propan-2-yl)amino)-4-oxobut anoic acid, N4-(1,3-bis(oleoyloxy)-2-((oleoyloxy)methyl)propan-2-yl)-L-asparagine, or N5-(1,3-bis(oleoyloxy)-2-((oleoyloxy)methyl)propan-2-yl)-L-glutamine.

7. An ionic neutral lipid comprising a Tris skeleton, wherein
a fatty acid residue is bonded to each of three oxygen atoms of the Tris skeleton, the fatty acid residue has 1 to 22 carbon atoms in a hydrocarbon chain moiety thereof and may have one or more unsaturated bonds, and the three fatty acid residues in one molecule may be the same group or two or more different groups, and
an anionic group is linked to one nitrogen atom of the Tris skeleton via a divalent linking group having an ammonium cationic group or a quaternary ammonium cationic nitrogen atom.

8. A lipid nanoparticle comprising the ionic neutral lipid according to any one of claims 1 to 7.

9. The lipid nanoparticle according to claim 8, further comprising a sterol and a polyalkylene glycol-modified lipid.

10. The lipid nanoparticle according to claim 8, further comprising a pH-sensitive cationic lipid having a pKa of 6.6 or less.

11. The lipid nanoparticle according to claim 8, containing a nucleic acid.

12. The lipid nanoparticle according to claim 11, wherein the nucleic acid is siRNA, mRNA, or plasmid DNA.

13. A pharmaceutical composition comprising the lipid nanoparticle containing the ionic neutral lipid according to any one of claims 1 to 7 as an active ingredient.

14. A pharmaceutical composition comprising, as an active ingredient, the lipid nanoparticle containing the ionic neutral lipid according to any one of claims 1 to 7 and a nucleic acid.

15. The pharmaceutical composition according to claim 14, which is used for gene therapy.

16. A method for expressing a foreign gene, comprising administering to a subject animal (excluding human), the lipid nanoparticle according to claim 11, which encapsulates a foreign gene of interest to be expressed in a target cell, and expressing the foreign gene in the target cell of the subject animal.
